# EUROPEAN PATENT SPECIFICATION

(11) **EP 1 263 958 B1**
(45) Date of publication and mention of the grant of the patent: **21.11.2007**
(21) Application number: 01959926.5
(22) Date of filing: 16.03.2001
(51) Int. Cl.: C12N 15/12, C07K 14/47, C12N 15/10, C12N 15/62, C12Q 1/68, A01K 67/027, C07K 16/18, A61K 31/7105, A61K 31/59

(54) **NUSAP, A GENE ENCODING A TISSUE-SPECIFIC NUCLEAR PROTEIN, USEFUL AS A DIAGNOSTIC TOOL AND THERAPEUTIC AGENT**
NUSAP, EIN GEN, WELCHES FÜR EIN GEWEBE-SPEZIFISCHES PROTEIN KODIERT, VERWENDUNG ALS DIAGNOSEMITTEL UND THERAPEUTISCHE ANWENDUNG
NUSAP, NOUVEAU GENE CODANT UNE PROTEINE NUCLEAIRE DE SPECIFICITE TISSULAIRE UTILE EN TANT QU'OUTIL DE DIAGNOSTIC ET AGENT THERAPEUTIQUE

(30) Priority: 16.03.2000 US 189743 P
(43) Date of publication of application: 11.12.2002
(73) Proprietor: K.U. LEUVEN RESEARCH & DEVELOPMENT, 3000 Leuven (BE)
(72) Inventor: BOUILLON, Roger, B-3020 Herent (Winksele) (BE); CARMELIET, Geert, B-3052 Oud-Heverlee (BE); RAEMAEKERS, Tim, B-3500 Hasselt (BE)
(74) Representative: Bird, Ariane
(86) International application number: PCT/EP2001/002971
(87) International publication number: WO 2001/070798

(56) References cited:
- WO-A-00/55633
- WO-A-01/70808
- DATABASE EMBL [Online] Accession AU080124, 19 October 1999 (1999-10-19) HASHIMOTO K ET AL: "Mus musculus brain cDNA, clone:MNCb-5258, 5' end." XP002187276
- DATABASE EMBL [Online] Accession AV117192, 25 June 1999 (1999-06-25) ARAKAWA T ET AL: "Mus musculus 10 days embryo cDNA, RIKEN full-length enriched library, clone:2610201A12, 3' end partial sequence." XP002187277
- DATABASE EMBL [Online] accession AF090915, 14 January 2000 (2000-01-14) YU Y ET AL: "Homo sapiens clone HQ0310 PRO0310p1 mRNA, complete cds." XP002187278
- DATABASE EMBL [Online] Accession AF305710, 11 November 2000 (2000-11-11) SATO H ET AL: "Mus musculus nucleolar protein ANKT mRNA, complete cds." XP002187279
- DATABASE EMBL [Online] Accession AF305711, 25 October 2000 (2000-10-25) SATO H ET AL: "Homo sapiens nucleolar protein ANKT mRNA, complete cds." XP002187280
- SEGAERT S ET AL: CURRENT OPINION IN CLINICAL NUTRITION AND METABOLIC CARE, vol. 1, no. 4, 1998, pages 347-354, XP001042314

## Description

### FIELD OF THE INVENTION

This invention relates to a gene which is related to DNA replication, cell proliferation and meiosis and has a discrete localisation in the cell nucleus. This invention further relates to the use of polynucleotide and amino acid sequences in the diagnosis, prevention, and treatment of disorders associated with DNA replication, cell proliferation and meiosis, such as cancer, hyperproliferation of cells, disorders of reproduction in eukaryotic cells, disorders of the immune system and disorders related to bones of mammals.

### BACKGROUND OF THE INVENTION

The biologically active form of vitamin D₃, 1α,25-dihydroxyvitamin D₃ [1α,25(OH)₂D₃], has an important role in bone and mineral homeostasis (Bouillon et al. in Endocr. Rev. (1995) 16:200-257). Most actions of 1α,25(OH)₂D₃ are mediated by binding to the vitamin D receptor, a nuclear receptor that functions as a ligand-induced transcription factor that modulates gene expression (Mangelsdorf et al. in Cell (1995) 83:835-839). *In vitro* experiments have shown that 1α,25(OH)₂D₃ can stimulate or inhibit the differentiation of osteoblasts, the bone-forming cells, depending whether it is given during the proliferation or differentiation phase respectively (Owen et al. in Endocrinology (1991) 128:1496-1504). The pro-differentiating effect of 1α,25(OH)₂D₃ is reflected by the induction of gene expression of osteocalcin, osteopontin and alkaline phosphatase which are characteristic for osteoblast differentiation. Vitamin D responsive elements were identified in several of these proteins (Haussler et al. in J. Bone Miner. Res. (1998) 13:325-349). However, the transcriptional control by 1 α,25(OH)₂D₃ is more complex and is likely to be modulated by nuclear matrix- and/ or chromatin-factors (Stein et al. in Physiol. Rev. (1996) 76:593-629). In osteoblasts, for example, the vitamin D responsiveness of the osteocalcin gene is altered by interaction with YY1, an ubiquitous transcription factor which localizes predominantly in the nucleolus and also associates with the nuclear matrix (Guo et al. in Proc. Natl. Acad. Sci. USA. (1997) 94:121-126). Moreover, impaired transcriptional control by the glucocorticoid receptor, another steroid receptor, has been observed after deletion of the chromosomal protein HMG1 (Calogero et al. in Nat. Genet. (1999) 22:276-280).

The effect of 1α,25(OH)₂D₃ on the proliferation of osteoblasts and the underlying mechanisms remain however largely unknown. Progression through the cell cycle is in part regulated by the activation and inactivation of cyclin-dependent kinase complexes at specific time points. 1α,25(OH)₂D₃ has the capacity to act as an inhibitor of cell growth of several cancer cells and keratinocytes by blocking the transition from the G1 to the S phase of the cell cycle (Walters in Endocr. Rev. (1992) 13:719-764; Bouillon et al. cited *supra*). Its coordinate induction of several cyclin-dependent kinase inhibitors, including p21 and p27 either directly or indirectly (Liu et al. in Genes Dev. (1996) 10:142-153; Segaert et al. in J. Invest. Dermatol. (1997) 109:46-54), together with altered cyclin levels (Wang et al. in Cancer Res. (1996) 56:264-247; Verlinden et al. in Mol. Cell. Endocrinol. (1998) 142:57-65) after treatment have been suggested to cooperatively contribute to cell cycle arrest.

Cell proliferation is normally accompanied by the faithful transmission of genetic information. This requires accurate replication and repair of genomic DNA during the Sand G2-phase of the cell cycle. Then, during the mitotic phase, the chromosomes segregate and this involves a coordinated sequence of structural changes including nuclear envelope breakdown, chromosome condensation, collapse of the cytoskeleton and formation of the mitotic spindle (Mc Intosh in Cold Spring Harb. Symp. Quant. Biol. (1991) 56:613-619). It remains unknown until now whether 1α,25(OH)₂D₃ has an effect on the expression of genes involved in one of these processes.

There is a continuous need, with respect to disorders associated with DNA replication, cell proliferation and meiosis, such as cancer, hyperproliferation of cells, disorders of reproduction in eukaryotic cells, disorders of the immune system and disorders related to bones of mammals, to identify and elucidate new proteins having a role in fundamental processes involved in such diseases. In particular, there is a continuous need to identify proteins, the expression level or malfunctioning of which may have a causative role in such diseases, and/or which may be used as diagnostic tools for such diseases. More specifically, since - as described above - vitamin D is an important regulator of bone cell differentiation, a problem addressed by the present invention is the identification of a protein, the expression of which can be modulated by vitamin D supply to osteoblast cells.

### SUMMARY OF THE INVENTION

The present invention is based on the unexpected discovery of a novel Nuclear Spindle-Associating Protein and the associated polynucleotide and at least some of their relevant biological activities.

The present invention therefore first provides an isolated and purified eukaryotic polynucleotide encoding a Nuclear Spindle-Associating Protein (hereinafter referred as NuSAP), the said polynucleotide (hereinafter referred as *nusap*) containing the nucleic acid sequence shown in SEQ ID NO: 1 (encoding for mouse NuSAP) or the nucleic acid sequence shown in SEQ ID NO:3 (encoding for human NuSAP), or an allelic variant thereof.
The present invention also includes a protein having the amino acid sequence shown in SEQ ID NO:2 (mouse NuSAP) or the amino acid sequence SEQ ID NO:4 (human NuSAP) or a protein having at least 90 % amino acid identity to said protein having SEQ ID NO:2 or SEQ ID NO:4 or a protein encoded by an allelic variant of a polynucleotide containing the nucleic acid sequence shown in SEQ ID NO: 1 or the nucleic acid sequence shown in SEQ ID NO: 3. The present invention also includes a eukaryotic polynucleotide as defined above, further comprising a polynucleotide which codes for at least a portion of a protein or a marker, the combination of both polynucleotides being arranged in such a way that a fusion protein results after expression.

The present invention further includes a vector, for instance an expression vector or a non-expression vector, comprising at least a eukaryotic polynucleotide as defined above.

The present invention also includes a fusion protein resulting after expression of the combination of a eukaryotic polynucleotide as defined above and a further polynucleotide coding for at least a portion of a protein or a marker.

The present invention further includes a eukaryotic polynucleotide containing the nucleic acid sequence shown in SEQ ID NO:1 or the nucleic acid sequence shown in SEQ ID NO:3, or an allelic variant thereof, for use as a medicament.

The present invention also includes the use of a eukaryotic polynucleotide such as defined herein-above for the manufacture of a medicament, in particular for therapeutic treatment or prevention in mammals. In a particular embodiment, said medicament is for the prevention or treatment of a disorder associated with DNA replication, cell proliferation or meiosis. In further particular embodiments, the disorder is a disorder associated with cell hyperproliferation. In further particular embodiments, the disorder is a disorder caused by mutations in nuclear structural proteins, such as Emery-Dreifuss muscular distrophy, dilated cardiomyopathy or lipodistrophy. In further particular embodiments, the disorder is cancer, most particularly a cancer of the tissue selected from the group consisting of kidney, stomach, small intestine, lung, ovary, cervix, breast and uterus.

In particular embodiments, the disorder is a disorder of reproduction in eukaryotic cells. In further particular embodiments, the disorder is a disorder of the immune system. In further particular embodiments, the disorder is a disorder related to bones of mammals.

The present invention also includes the use of a eukaryotic polynucleotide containing the nucleic acid sequence shown in SEQ ID NO:1 or in SEQ ID NO:3 or an allelic variant thereof, or of a probe capable of specifically hybridizing with, or specifically amplifying a eukaryotic polynucleotide containing the nucleic acid sequence shown in SEQ ID NO: 1 or in SEQ ID NO:3 or of an allelic variant thereof, for the manufacture of a diagnostic tool, more particularly for the detection of cancer of a tissue selected from the group consisting of kidney, stomach, small intestine, lung ovary, cervix, breast and uterus.

The present invention includes the use of a protein having the amino acid sequence shown in SEQ ID NO:2 or SEQ ID NO:4 or of a protein having at least 90 % amino acid identity to said protein having SEQ ID NO:2 or SEQ ID NO:4 or of a protein encoded by an allelic variant of a polynucleotide containing the nucleic acid sequence shown in SEQ ID NO: 1 or the nucleic acid sequence shown in SEQ ID NO: 3, or of a portion thereof, or of a fusion protein as defined above, for the manufacture of a diagnostic tool, more particularly for the detection of cancer of a tissue selected from the group consisting of kidney, stomach, small intestine, lung ovary, cervix, breast and uterus.

The present invention also includes the use of a fusion protein of a eukaryotic polynucleotide containing the nucleic acid sequence shown in SEQ ID NO:1 or the nucleic acid sequence shown in SEQ ID NO:3, or an allelic variant thereof as defined above, for the manufacture of a diagnostic tool, more particularly for the detection of cancer of a tissue selected from the group consisting of kidney, stomach, small intestine, lung ovary, cervix, breast and uterus. The present invention includes a pharmaceutical composition comprising a eukaryotic polynucleotide containing the nucleic acid sequence shown in SEQ ID NO:1 or the nucleic acid sequence shown in SEQ ID NO:3, or an allelic variant thereof and a pharmaceutically acceptable carrier.

The present invention also includes a process for the production of a protein having the amino acid sequence SEQ ID NO:2 or the amino acid sequence SEQ ID NO:4, or a protein having at least 90 % amino acid sequence identity to said protein having the amino acid sequence of SEQ ID NO:2 or SEQ ID NO 4 or a protein encoded by an allelic variant of a polynucleotide containing the nucleic acid sequence shown in SEQ ID NO: 1 or the nucleic acid sequence shown in SEQ ID NO:3., comprising culturing a host cell containing an expression vector comprising the nucleic acid sequence shown in SEQ ID NO: 1 or the nucleic acid sequence shown in SEQ ID NO:3, or an allelic variant thereof, and obtaining said protein from the cell or the culture medium.

The present invention further includes a host cell, other than a human germ line cell, being transformed with a eukaryotic polynucleotide comprising the nucleic acid sequence shown in SEQ ID NO:1 or the nucleic acid sequence shown in SEQ ID NO:3, or an allelic variant thereof, optionally further comprising a polynucleotide which codes for at least a portion of a protein or a marker, the combination of both polynucleotides being arranged in such a way that the fusion protein results after expression.

The present invention further includes a transgenic rodent whose genome is heterozygous for an engineered disruption in a rodent NuSAP gene coding for SEQ ID NO: 1 or its rodent homologue, wherein said engineered disruption in a heterozygous state inhibits overall or tissue specific production of a NuSAP protein having the amino acid sequence SEQ ID NO:2 or the rodent homologue thereof, thereby resulting in a transgenic rodent which has increased tendency, with respect to a wild-type rodent, to disorders associated with DNA replication, cell proliferation and meiosis, such as cancer, hyperproliferation of cells, disorders of reproduction, disorders of the immune system and disorders related to bones. In a particular embodiment, said transgenic rodent further comprises a promoter in front of the gene for modulating the expression of the protein in comparison with the wild-type.

The present invention further includes a ribozyme capable of cleaving the mRNA of a NuSAP protein having the amino acid sequence SEQ ID NO:2 or the amino acid sequence SEQ ID NO:4, or a protein having at least 90 % amino acid sequence identity to said protein having the amino acid sequence of SEQ ID NO:2 or SEQ ID NO 4 or a protein encoded by an allelic variant of a polynucleotide containing the nucleic acid sequence shown in SEQ ID NO: 1 or the nucleic acid sequence shown in SEQ ID NO: 3, the said ribozyme including sequences complementary to portions of mRNA obtained from the polynucleotides, containing the nucleic acid sequence shown in SEQ ID NO: 1 or the nucleic acid sequence shown in SEQ ID NO:3, or an allelic variant thereof.

The present invention further includes an antisense oligonucleotide capable of blocking expression of an above-described eukaryotic polynucleotide containing the nucleic acid sequence shown in SEQ ID NO:1 or the nucleic acid sequence shown in SEQ ID NO:3, or an allelic variant thereof.

The present invention further includes a vector which produces in a cell, other than a human germ line cell, the above-described ribozyme or antisense oligonucleotide.

The present invention also includes a host cell, other than a human germ line cell, which is transformed with a polynucleotide containing the nucleic acid sequence shown in SEQ ID NO:1 or the nucleic acid sequence shown in SEQ ID NO:3, or an allelic variant thereof optionally further comprising a polynucleotide which codes for at least a portion of a protein or a marker, the combination of both polynucleotides being arranged in such a way that a fusion protein results after expression. The host cell may include a bacterium, fungus, plant or animal cell.

The present invention further provides a monoclonal antibody, being able to discriminate between a NuSAP Protein with and without a post-translationally modified amino-acid wherein the protein has the amino acid sequence SEQ ID NO:2 or the amino acid sequence SEQ ID NO:4, or has at least 90 % amino acid sequence identity to a protein having the amino acid sequence of SEQ ID NO:2 or SEQ ID NO 4 or wherein the protein is encoded by an allelic variant of a polynucleotide containing the nucleic acid sequence shown in SEQ ID NO: 1 or the nucleic acid sequence shown in SEQ ID NO:3.

### DESCRIPTION OF THE DRAWINGS

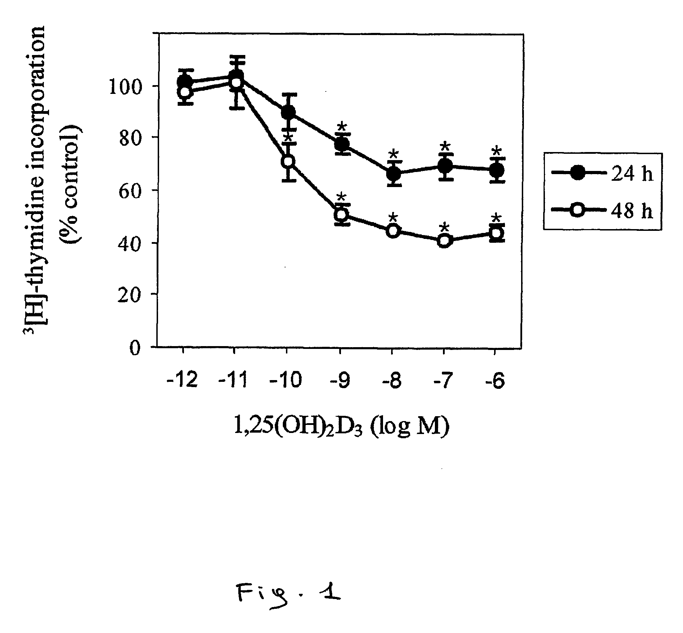

Figure 1 shows the identification of a novel 1,25(OH)₂D₃ regulated gene in proliferating mouse osteoblastic MC3T3-E1 cells. (a) The proliferation of MC3T3-E1 cells was analyzed after treatment for 24 and 48 h with varying doses of 1,25(OH)₂D₃ by monitoring [³H]-thymidine incorporation. Data represent mean ± SD of six replicates of a representative experiment. Values of vehicle (ethanol) treated cells were taken as 100 %. (**P* < 0.001; versus vehicle, *t* test)

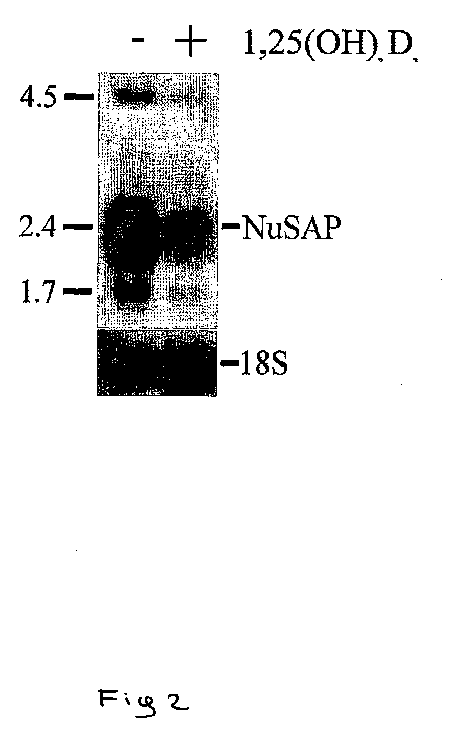

Figure 2 represents a Northern blot analysis showing *nusap* gene expression in sub-confluent cultures of MC3T3-E1 cells after treatment with vehicle or 1,25(OH)₂D₃ (10⁻⁸ M) for 24 hours. The size (kb) of the three transcripts is indicated.

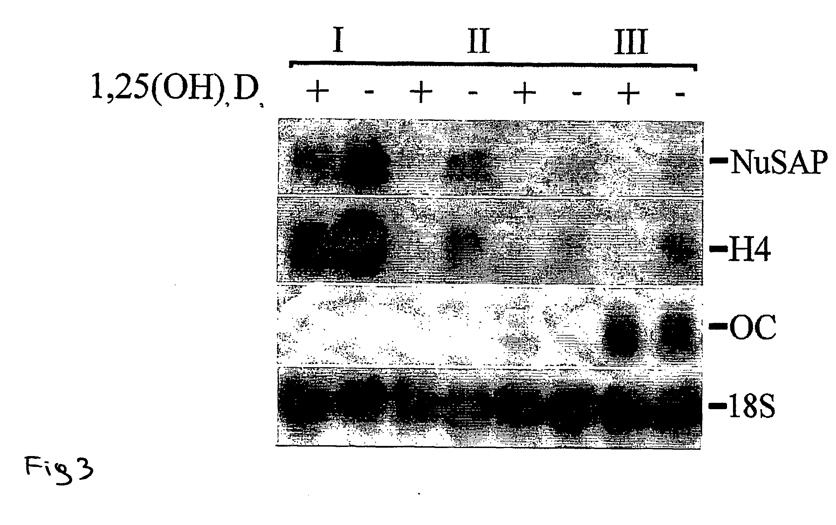

Figure 3 represents a Northern blot analysis showing *nusap* gene expression relative to growth markers (histone H4) and differentiation markers (osteocalcin) in developing MC3T3-E1 cells. Cells were cultured for 4 weeks during which they proceeded through subsequent stages of development: proliferation (I), matrix maturation (II), and mineralization (III). The regulation of gene expression of the different stage-specific markers by 1,25(OH)₂D₃ (10⁻⁸ M for 24 hours) is also shown. Subsequent hybridizations were performed with the following probes: human histone H4, pFO002; mouse osteocalcin , p923; and a 0.8 kb NuSAP BglII/ AspI restriction fragment. The 18S cDNA probe was used as a control to assess equal loading.

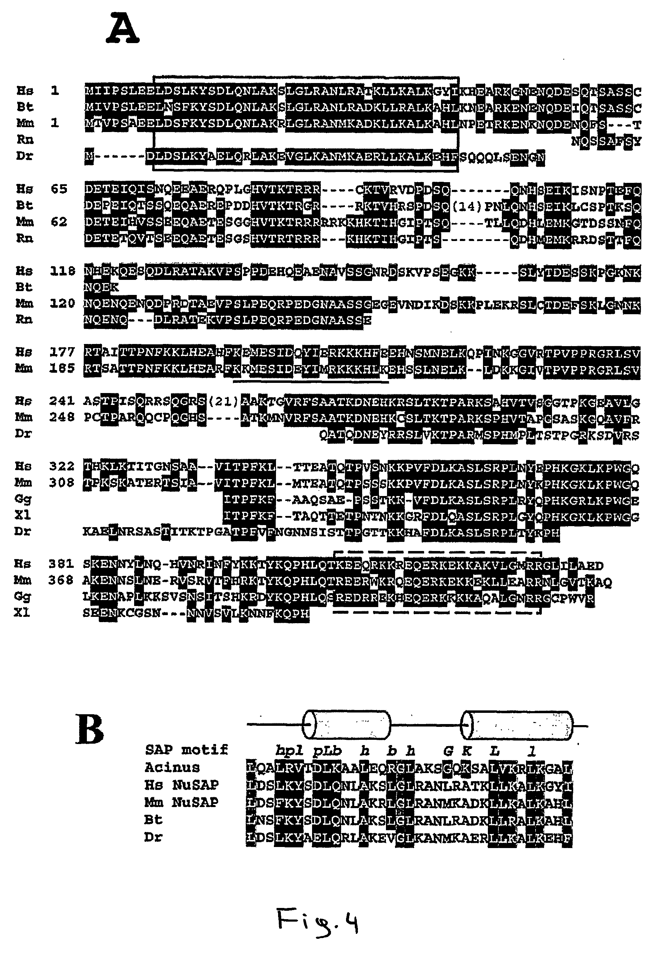

Figure 4 shows the deduced amino acid sequence of mouse and human NuSAP and its alignment with predicted proteins from other species (A), and with the SAP motif consensus sequence (B). (A) Identical and similar residues are shaded in black. Subgroups of homologous residues were defined as follows: positively charged residues (R,K), negatively charged residues (E,D), and hydrophobic residues (L,V,I,F,M). Gaps, indicated by dashes or numbers between parenthesis, were introduced for optimal alignment. Boxed at the N-terminus is the potential SAP motif, and at the C-terminus (in dashed lines) is a conserved stretch of charged residues that may be important for electrostatic interactions. Underlined is the potential nuclear localization signal identified in the mouse sequence. (B) Residues within the SAP motif consensus sequence have been defined by Aravind et al. in Trends Biochem. Sci. (2000) 25:112-4 as h (hydrophobic), *p* (polar), I (aliphatic), and b (bulky). Also shown is the sequence of mouse *AcinusL* (Accession No. AAF89661), a SAP module containing protein. Shaded in black are residues which agree with the consensus sequence, and in grey are residues which conform to the similarity as described in (A). Sequences besides those of mouse and human, were deduced from expressed sequence tags. The Genbank TM accession numbers are as follows: Hs, *Homo sapiens* (human) (AAG25874); Bt, *Bos taurus* (cattle) (BE480183); Mm, *Mus musculus* (mouse) (AAG31285); Rn, *Rattus norvegicus* (rat) (AA923940); Gg, *Gallus gallus* (chicken) (AJ392813); X1, *Xenopus laevis* (clawed frog) (AW642384); and Dr, *Danio rerio* (zebrafish) (AI545826, AI958745).

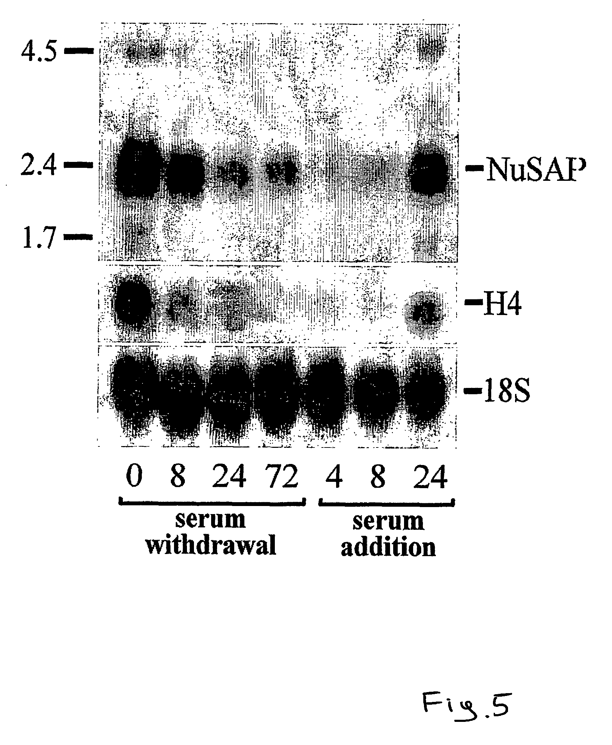

Figure 5 shows expression of the *nusap* gene in sub-confluent MC3T3-E1 cell cultures after serum withdrawal. Northern blot analysis of *nusap* expression after serum withdrawal (0.1 % fetal calf serum FCS) and upon serum re-addition (10 % FCS). Cells were harvested at the indicated time points (hours). The different cell cycle phases were assigned based on the RNA levels of *histone H4,* a S-phase marker. The blots were hybridized with the following cDNA probes: human histone H4, pFO002; and a 0.8 kb NuSAP BglII/ AspI restriction fragment. The 18S cDNA probe was used as a control to assess equal loading.

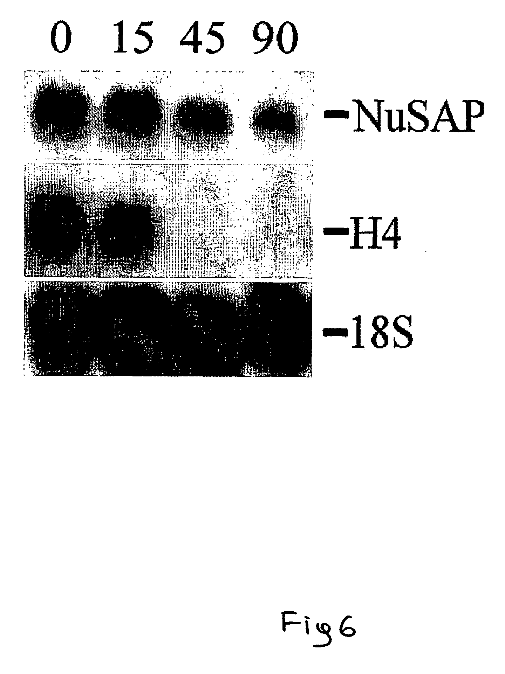

Figure 6 shows expression of the *nusap* gene in sub-confluent MC3T3-E1 cell cultures after hydroxyurea treatment. Northern blot analysis of *nusap* expression in hydroxyurea treated (1mM) MC3T3-E1 cells. Cells were harvested at the indicated time points (minutes) after treatment. The different cell cycle phases were assigned based on the RNA levels of *histone H4,* a S-phase marker. The blots were hybridized with the following cDNA probes: human histone H4, pFO002; and a 0.8 kb NuSAP BglII/ AspI restriction fragment. The 18S cDNA probe was used as a control to assess equal loading.

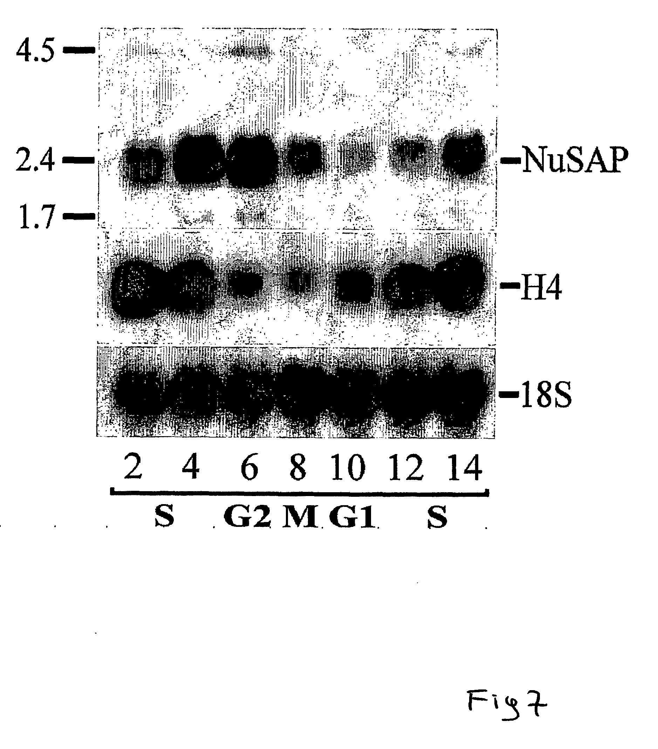

Figure 7 shows expression of the *nusap* gene in synchronised sub-confluent MC3T3-E1 cell cultures. Northern blot analysis of *nusap* expression in MC3T3-E1 cells synchronized using a double thymidine block. Cells were washed and released in growth medium and harvested at the indicated time points (hours). The different cell cycle phases were assigned based on the RNA levels of *histone H4,* a S-phase marker. The blots were hybridized with the following cDNA probes: human histone H4, pFO002; and a 0.8 kb NuSAP BgIII/ AspI restriction fragment. The 18S cDNA probe was used as a control to assess equal loading.

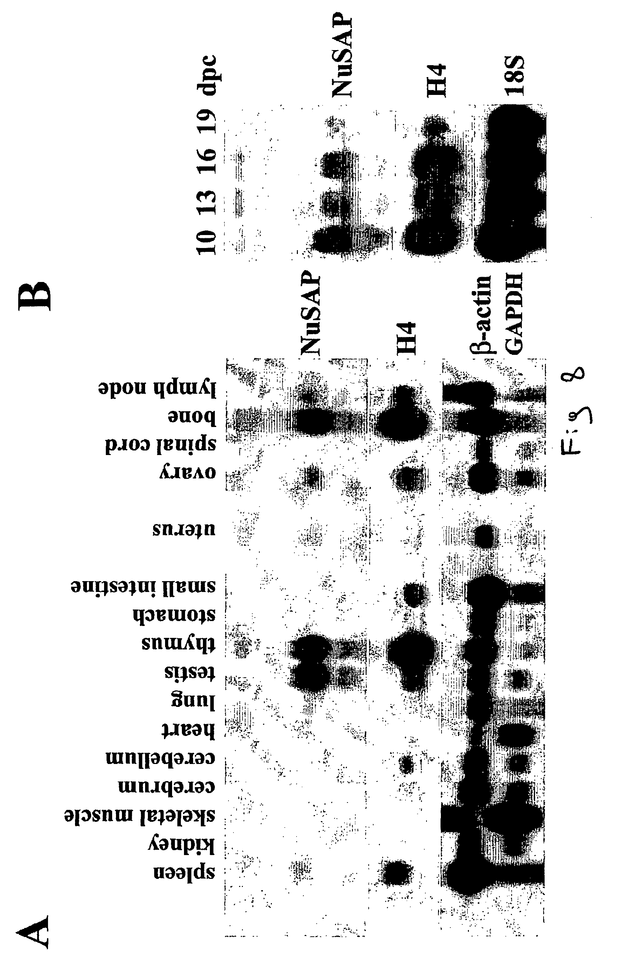

Figure 8 shows expression of *nusap* in mouse (C57BL/6) tissues and embryo. (a) Northern blot analysis of *nusap* expression in tissues from 2 months old mice. (b) Northern blot analysis of *nusap* expression in whole embryo's from different developmental stages (days post-coitum). The following cDNA probes were used: human histone H4 (pFO002), and a 0.8 kb NuSAP BglII/ AspI restriction fragment. To assess equal loading 18S, □-actin, and glyceraldehyde 3-phosphate dehydrogenase (GAPDH) cDNA probes were used.

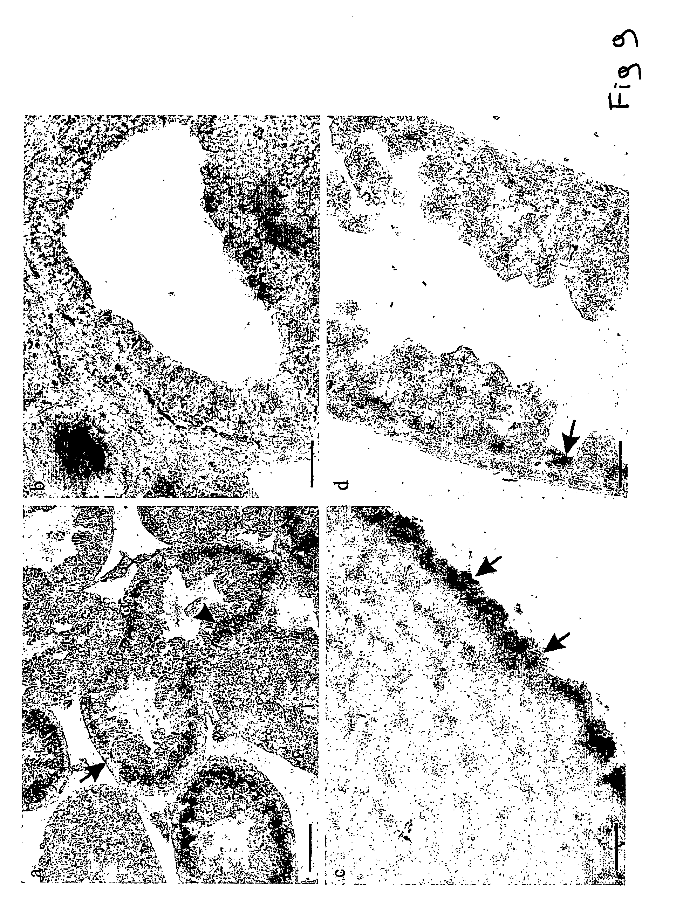

Figure 9 shows in situ hybridization of *nusap* in mouse tissues (C57BL/6). Sections were hybridized with a DIG-labeled *nusap* probe. (a) In sections of the adult testis, the primary spermatocytes (arrowhead) were stained positively. Spermatogonia, denoted by an arrow, were negative. (b) Expression of *nusap* in adult ovary sections was strong in oocytes (star). (c) In sections of the adult thymus, the subcapsular outer cortex region (arrows) showed positive staining. (d) Expression of *nusap* RNA in sections of the small intestine of a 19 days post-coitum embryo was intense in cells in the crypts of the villi (arrow). Bars: (a, d) 50 µm; (b, c) 30 µm.

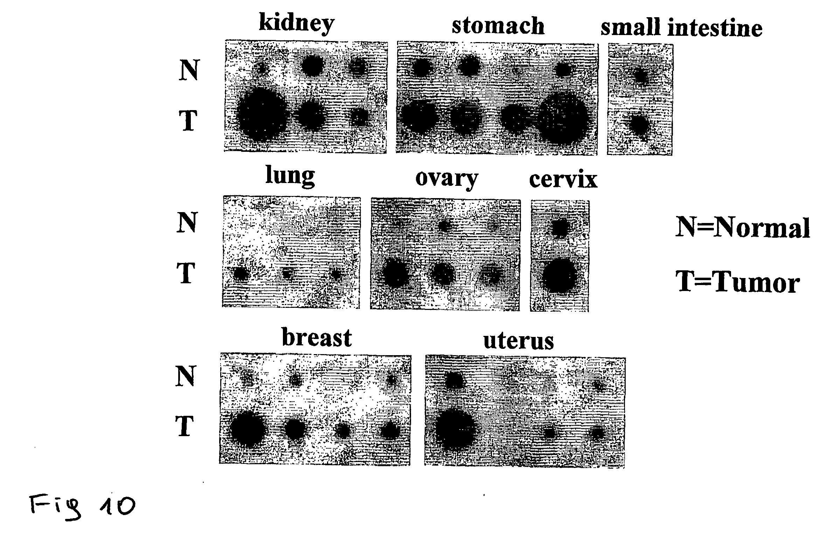

Figure 10 shows expression of nusap in tumor tissues. Elevated expression levels of NuSAP in cDNA pools of human tumor tissues (designated as T) is compared to the expression of NuSAP in cDNA pools obtained from the same tissue from healthy individuals (designated as N). Quantitive detecture of *nusap* is detected by exposure on a phosphorimager.

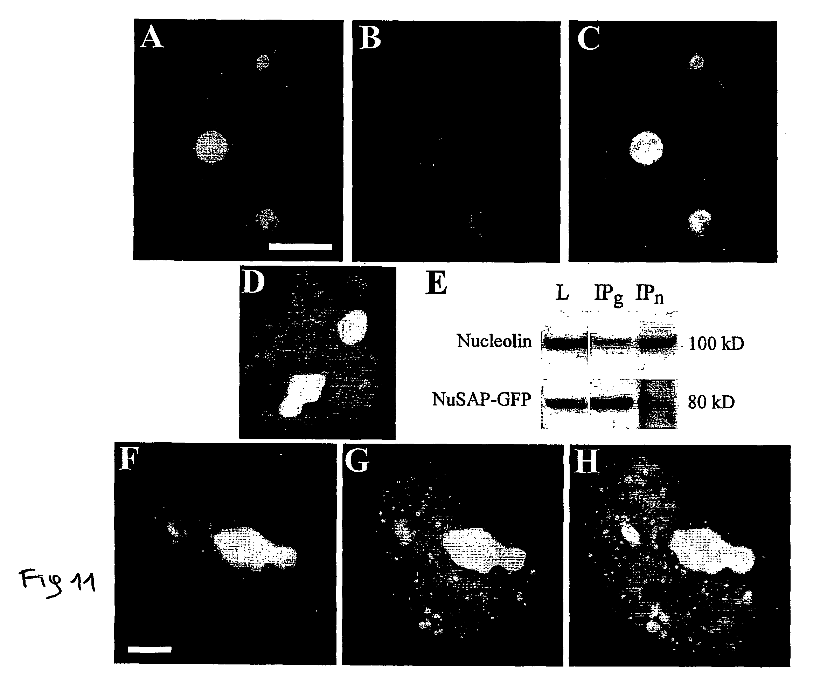

Figure 11 shows that NuSAP localizes to the nucleolus and to intranuclear filaments. Indirect immunofluorescence analysis of transfected COS-1 cells by confocal microscopy, shows GFP-tagged NuSAP localizing to the nucleus and to the nucleolus (A, D)(Green) which is stained for nucleolin (B)(red) respectively. C is the merger of A and B. Interaction between NuSAP-GFP and nucleolin was shown by immunoprecipitating COS-1 cell lysates (L) with antibodies to GFP (IPg) and to nucleolin (IPn) followed by Western blot analysis with antibodies to nucleolin and GFP (E). Consecutive sections taken at 1 µm z-axis increments show that GFP-tagged NuSAP localizes to an intranuclear filamentous network at the nuclear periphery of transfected COS-1 cells (F and G). At the core of the nucleus a more diffuse fluorescence pattern is observed (H). (Bar: 5 µm)

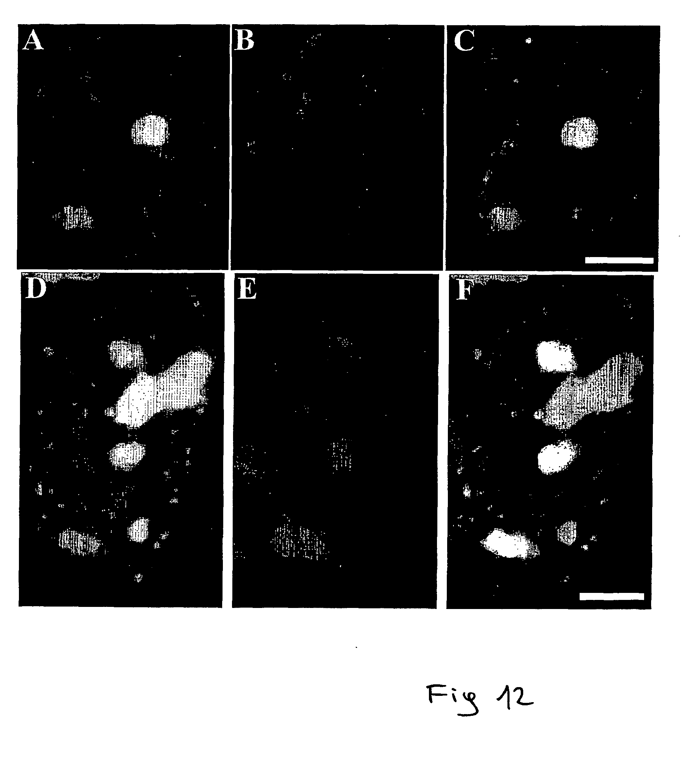

Figure 12 shows that transfected NuSAP localizes to sites of DNA replication in S-phase cells. COS-1 cells were transiently transfected with GFP-tagged NuSAP and incubated in medium containing BrdU (10 µM) for 30 minutes before fixation in 70 % ethanol. Indirect immunofluorescence analysis of transfected COS-1 cells in S-phase shows NuSAP-GFP foci (A) lying adjacent to sites of BrdUrd incorporation (B)(red). C is the merger of A and B. In some cells, BrdUrd incorporation is distinctly aggregated (E), and these sites overlap with NuSAP-GFP (D) and F (which is the merger of D and E).

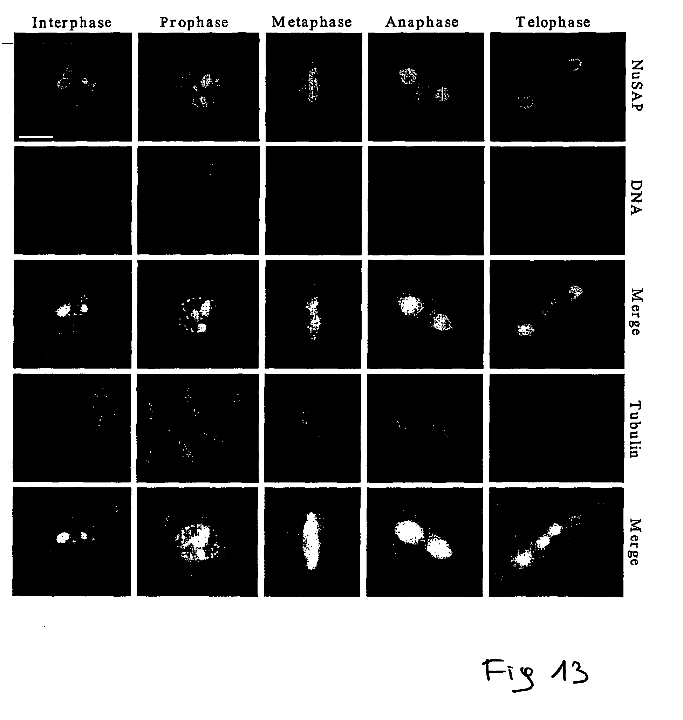

Figure 13 shows that during mitosis transfected NuSAP localizes to the chromosomes and microtubules. NuSAP expressing cells were examined for GFP fluorescence (green) and indirect immunofluorescence labeling of α-tubulin (red). DNA was stained with Hoechst 33258 (blue). (A) Assignment of the different stages of mitosis was based on morphological features. Fluorescence derived from GFP was merged with that derived from the DNA stain (middle merger) or from anti-α-tubulin staining (bottom merger). Bar: 15 µm.

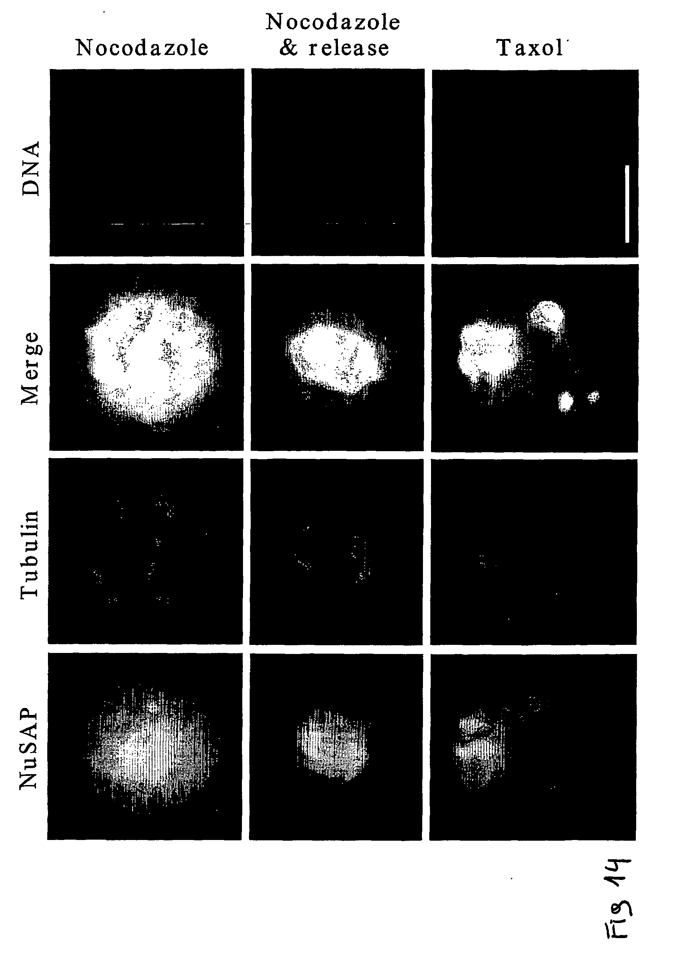

Figure 14 shows the localization of transfected NuSAP after treatment with nocodazole and taxol. Cells in mitosis were either treated with nocodazole for 1 hour (10 µM), or treated similarly and then washed and released in growth medium for 1 hour. Taxol treatment (5 µM for 1 hour) induced the formation of microtubule asters. NuSAP-expressing cells were examined for GFP fluorescence (green) and indirect immunofluorescence labeling of α -tubulin (red). Merge is an overlay oftubilin and NuSAP-GFP. DNA is detected by Hoechst staining (blue). Bar: 10 µm.

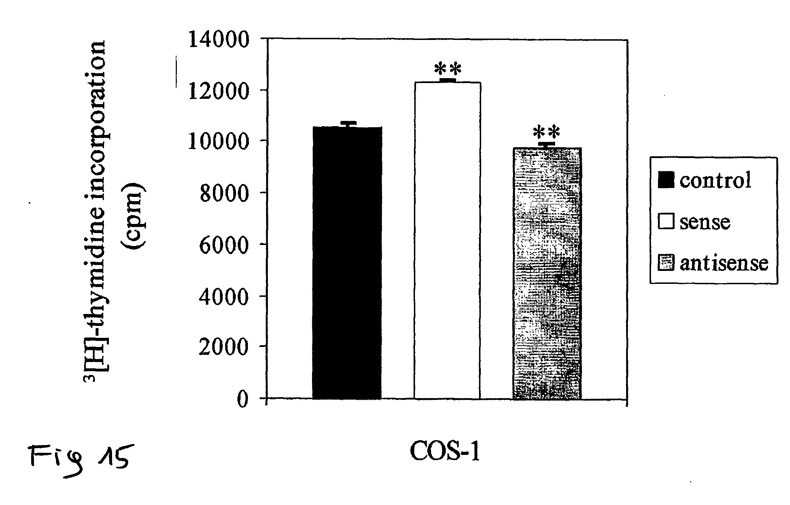

Figure 15 shows the overexpression of sense and antisense NuSAP in COS-1 cells. COS-1 cells were transiently transfected with sense and antisense constructs of NuSAP. As a control, a vector without insert was used. Cell proliferation was analyzed 48 hours post-transfection and monitored by [³H]-thymidine incorporation. Data represent mean ± standard deviation of six replicates of a representative experiment. (***P* < 0.01, **P* < 0.05; versus control, *t* test).

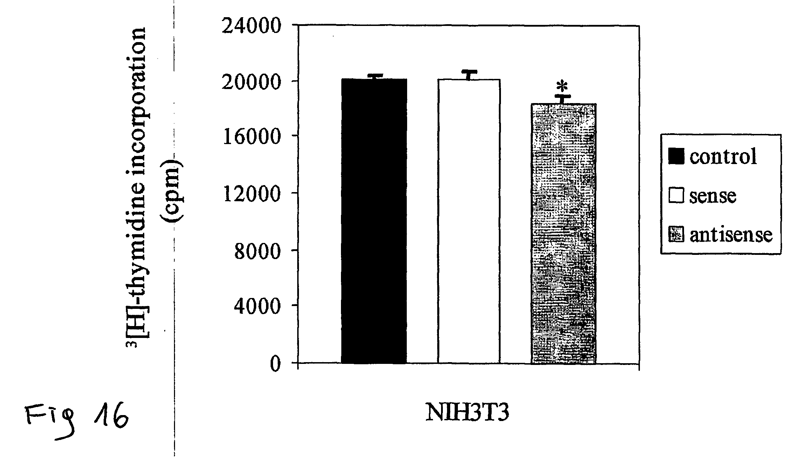

Figure 16 shows overexpression of sense and antisense NuSAP in NIH3T3 cells. NIH3T3 cells were transiently transfected with sense and antisense constructs of NuSAP. As a control, a vector without insert was used. Cell proliferation was analyzed 48 hours post-transfection and monitored by [³H]-thymidine incorporation .Data represent mean ± standard deviation of six replicates of a representative experiment. (***P* < 0.01, **P* < 0.05; versus control, *t* test).

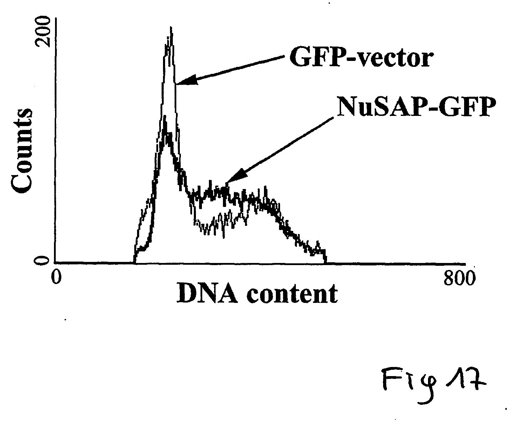

Figure 17 represents cell cycle profile of COS-1 cells 48 hours post transfection, showing a clear increase of S-phase population in cells overexpressing GFP-tagged NuSAP, relative to the control (GFP-vector).

### DEFINITIONS

The term "hybridization", as used herein, refers to any process by which a strand of nucleic acid binds with a complementary strand through base pairing.

The term "complementary", as used herein, refers to the natural binding of polynucleotides under permissive salt and temperature conditions by base pairing. For example, the sequence 5'-AGTT-3' binds to the complementary sequence 5'-AACT-3'. Complementarity between two single-stranded molecules may be "partial", in which only some of the nucleic acids bind, or it may be "complete" when total complementarity exists between the single stranded molecules. The degree of complementarity between polynucleotides has significant effects on the efficiency and strength of hybridization between polynucleotides. This is of particular importance in amplification reactions, which depend upon binding between polynucleotides.

The term "homology" or "homologue", as used herein, refers to a degree of similarity between two sequences.

The term "stringent conditions", as used herein, is the stringency which occurs within a range from about Tm-5°C (5°C below the melting temperature of the probe) to about 20°C to 25°C below Tm. As will be understood by those skilled in the art, the stringency of hybridization may be altered to identify or detect identical or related polynucleotide sequences.

The term "antisense", as used herein, refers to nucleotide sequences which are complementary to a specific DNA or RNA sequence. The term "antisense strand" is used in reference to a nucleic acid strand that is complementary to the "sense" strand. Antisense molecules may be produced by any method, including synthesis by ligating the gene(s) or interest in a reverse orientation to a promoter which permits the synthesis of a complementary strand. Once introduced into a cell, this transcribed strand combines with natural sequences produced by the cell to form duplexes. These duplexes then block either the further transcription or translation. In this manner, mutant phenotypes may be generated.

The term "portion", as used herein, for instance with regard to a protein, refers to fragments thereof. The protein fragments may range in size from at least about 10 amino acid residues, preferably at least about 20 amino acid residues and more preferably at least about 40 amino acid residues, up to the entire amino acid sequence except for one amino acid. The same definition applies to nucleotide sequences as well, the fragments thereof may range in size from at least about 15 nucleotides, preferably at least about 30 nucleotides and more preferably at least about 60 nucleotides.

"Transformation", as defined herein, describes a process by which exogenous DNA enters and changes a recipient cell. It may occur under natural or artificial conditions using various methods well known in the art. Transformation may rely on any known method for the insertion of foreign nucleic acid sequences into a prokaryotic or eukaryotic host cell. The method is selected based on the host cell being transformed and may include, but is not limited to, viral infection, electroporation, lipofection, and particle bombardment. Such transformed cells include stably transformed cells in which the inserted DNA is capable of replication either as an autonomously replicating plasmid or as part of the host chromosome. They also include cells which transiently express the inserted DNA or RNA for limited periods of time.

As used herein, an "allele" or "allelic" sequence is an alternative form of the gene which may result from at least one mutation in the polynucleotide. Alleles may result in altered mRNAs or polypeptides whose structure or function may or may not be altered.

The term "intermediate", as used herein with respect to a NuSAP protein, means a messenger RNA, a sense RNA or an anti-sense RNA which eventually can lead to a Nuclear Spindle-Associating Protein, a homologue, a variant, a mutation or a portion thereof.

As used therein, the term "vitamin D" is defined according to the nomenclature of Vitamin D established by the International Union of Pure and Applied Chemistry and includes all vitamin D compounds and derivatives mentioned therein.

### DETAILED DESCRIPTION OF THE INVENTION

The present invention is based on the discovery of a new nuclear protein (NuSAP) involved in cell proliferation, eukaryotic polynucleotides encoding NuSAP and their use for the diagnosis, prevention and treatment of various diseases including various forms of cancer, hyperproliferation of cells and other disorders associated with cell proliferation and meiosis.

The present invention discloses eukaryotic polynucleotides encoding for NuSAP. The human (SEQ ID NO:3) and mouse (SEQ ID NO:1) polynucleotides of the novel gene *nusap* have been cloned. The chromosome localisation of the human *nusap* gene is on 15q15. The human and mouse sequences are more than 60 % identical at the amino acid level (SEQ ID NO:4 and SEQ ID NO:2 respectively).

Homologues of *nusap* are present in organisms other than mouse and man. It will be appreciated by those skilled in the art that sequences originating from these other organisms will differ to some extent from the mouse and human nucleic acid sequences specifically disclosed herein.

Also, it will be appreciated by those skilled in the art that, as a result of the degeneracy of the genetic code, a multitude of nucleotide sequences encoding NuSAP, some bearing low similarity to the nucleotide sequences of any known and naturally occurring gene, may be produced. Thus, this application contemplates each possible variation and/or portion of nucleotide sequence that could be made by selecting combinations based on possible codon choices.

The application also teaches the production of polynucleotide sequences, or portions thereof, which encode NuSAP and its derivatives, entirely by synthetic chemistry. Synthetic sequences may be used to introduce mutations into a sequence encoding NuSAP or any portion thereof. Such sequences may also be inserted in any available expression vector and cell system, while using reagents well known in the.

Altered polynucleotides encoding NuSAP that are disclosed in the application include deletions, insertions, or substitutions of different nucleotides resulting in a polynucleotide encoding a portion and/or a mutated version of NuSAP.

The polynucleotides encoding NuSAP may be extended by using a portion of the nucleotide sequence and employing various methods known in the art to detect upstream sequences such as promoters and regulatory elements. Such methods include among others "restriction-site" polymerase chain reaction (hereinafter referred as PCR), inverse PCR and capture PCR or chromosome walking techniques.

The invention encompassesNuSAP variants with at least 90 %, amino acid sequence identity to the NuSAP amino acid sequence.

The present application also discloses polynucleotide sequences that are capable of hybridizing to the claimed polynucleotide sequences, in particular those shown in SEQ ID NO:1 and SEQ ID NO:3, under various stringent conditions.

In another embodiment of the present invention, polynucleotide sequences containing the nucleic acid sequence shown in SEQ ID NO: 1 or the nucleic acid sequence shown in SEQ ID NO:3, or allelic variants thereof , or fusion proteins thereof, may be used in recombinant DNA molecules to direct expression of NuSAP in appropriate host cells. As will be understood by those skilled in the art, it may be advantageous to produce NuSAP-encoding polynucleotides possessing non-naturally occurring codons, for instance in order to increase the half-life of the transcript. The nucleotide sequences of the present invention can further be engineered using methods generally known in the art to modify the cloning, processing and/or expression of the gene product. Chimeric fusion proteins can be engineered to tag the protein an/or to enhance purification. A fusion protein in the latter case may be engineered to contain a cleavage site located between the NuSAP encoding sequence and the heterologous protein sequence.

In order to express biologically active NuSAP, the polynucleotide encoding NuSAP or a biological equivalent thereof may be inserted into an appropriate vector, i.e. a vector which contains the necessary elements for the transcription and translation of the inserted coding sequence. Methods used to construct such expression vectors are well known to those skilled in the art. Expression vectors derived from retroviruses, adenoviruses, herpes or vaccinia viruses or from various plasmids may be used for delivering the polynucleotides of this invention to the targeted organ tissue or cell population. Specific examples of such vectors include the pcDNA3.1(-)/Myc-His B plasmid, the pEGFP-N1 plasmid and the like. In order to express biologically active NuSAP, integration of the fused gene into the chromosomal DNA of a host may be considered as well.

A variety of expression vector/host systems may be utilized to contain and express sequences encoding NuSAP. Host cells can include, but are not limited to bacteria, yeast, insect, plant or animal cells. A host cell strain may be chosen to modulate the expressed protein in the desired fashion. For long-term, high-yield production of recombinant proteins, stable expression is preferred. Any number of selection system may be used to recover transformed cells. Alternatively, host cells which contain the polynucleotide encoding NuSAP and expressing NuSAP may be identified by a variety of procedures known to those skilled in the art, such as labels and conjugation techniques typically used in various nucleic acid and amino acid assays.

Host cells transformed with polynucleotides encoding NuSAP may be cultured under conditions suitable for the expression and recovery of the protein from the cell culture. The protein produced by a recombinant cell may be secreted or contained intracellularly depending on the sequence and/or the vector used. Expression vectors containing polynucleotides encoding NuSAP may be designed to contain signal sequences which direct secretion of NuSAP through a prokaryotic or eukaryotic membrane. Other recombinant constructions may be used to join sequences encoding NuSAP to polynucleotides encoding a polypeptide domain which will facilitate purification of soluble proteins. Such purification facilitating domains are well known in the art.

In one example it is shown that over-expression of antisense NuSAP inhibits cell proliferation. Antisense strands may be produced by any method, including synthesis by ligating the sequence of interest in a reverse orientation in an expression vector, which permits the synthesis of the complementary strand. Antisense molecules may be used to modulate NuSAP activity or to achieve regulation of the gene function. The disclosed polynucleotides encoding for NuSAP antisense strands, or a vector containing these sequences may for instance be administered to a mammal in order to prevent or treat a disorder associated with hyperproliferation of cells. Such disorders include various types of cancer. A composition comprising therapeutically effective amounts of antisense strands to a polynucleotide (RNA) encoding NuSAP may be mixed with any pharmaceutically acceptable carrier. The invention thus provides an antisense nucleic acid molecule that is capable of blocking the expression of the mRNA encoding a NuSAP protein having the amino acid sequence shown in SEQ ID NO:2 (mouse NuSAP) or the amino acid sequence SEQ ID NO:4 (human NuSAP) or a protein having at least 90 % amino acid identity to said protein having SEQ ID NO:2 or SEQ ID NO:4 or a protein encoded by an allelic variant of a polynucleotide containing the nucleic acid sequence shown in SEQ ID NO: 1 or the nucleic acid sequence shown in SEQ ID NO: 3. Antisense nucleic acid molecules can be RNA or single-stranded DNA, and can be complementary to the entire mRNA molecule encoding NuSAP or to only a portion thereof. These antisense molecules can be used to reduce levels of NuSAP, for instance by introducing into cells an RNA or single-stranded DNA molecule that is complementary to at least a portion of the mRNA of NuSAP (i.e. by introducing an antisense molecule). For a general discussion of antisense molecules and their use, reference is made to Rossi Br Med. Bull. (1995) 51:217-25.

The invention further provides a special category of antisense RNA molecules, known as ribozymes, having recognition sequences complementary to specific regions of the mRNA encoding the NuSAP protein having the amino acid sequence shown in SEQ ID NO:2 (mouse NuSAP) or the amino acid sequence SEQ ID NO:4 (human NuSAP) or a protein having at least 90 % amino acid identity to said protein having SEQ ID NO:2 or SEQ ID NO:4 or a protein encoded by an allelic variant of a polynucleotide containing the nucleic acid sequence shown in SEQ ID NO: 1 or the nucleic acid sequence shown in SEQ ID NO: 3. Ribozymes not only complex with target sequences via complementary antisense sequences but also catalyze the hydrolysis, or cleavage, of the template mRNA molecule.

Expression of a ribozyme in a cell can inhibit gene expression. More particularly, a ribozyme having a recognition sequence complementary to a region of a mRNA encoding NuSAP can be used to decrease expression of NuSAP. A vector may be used for introduction of the ribozyme into a cell. For a general discussion of ribozymes and their use, reference is made to Christoffersen. J. Med. Chem. (1995) 38:2023-37.

A possible alternative comprises the use of antibodies raised against NuSAP. A method for obtaining for instance mouse anti-human monoclonal antibodies is well known to those skilled in the art, including the humanization of antibodies by replacing amino acids in the non-antigen binding regions in order to more closely resemble a human antibody, while still retaining the original binding ability. Such a method is described for instance in Fundamental Immunology (1999), Lippincott-Raven. Protein biological activity is often regulated by post-translational modifications (hereinafter referred as PTM). Antibodies raised against NuSAP that can discriminate against a particular PTM (e.g. phosphorylation) are a useful tool in the diagnosis of a disease caused by a modified pattern compared to healthy individuals, independent from the fact whether the modified PTM is caused by a mutation in the protein itself or due to a mutation leading to a decreased function.

Pharmaceutically acceptable carriers as used throughout this application are well known to those skilled in the art and there is no particular restriction to their selection within the present invention. They may also include additives such as wetting agents, dispersing agents, stickers, adhesives, emulsifying agents, solvents, coatings, antibacterial and antifungal agents (for example phenol, sorbic acid, chlorobutanol), isotonic agents (such as sugars or sodium chloride) and the like, provided the same are consistent with pharmaceutical practice, i.e. carriers and additives which do not create permanent damage to mammals. The pharmaceutical compositions of the present invention may be prepared in any known manner, for instance by homogeneously mixing, coating and/or grinding the active ingredients, in a one-step or multi-steps procedure, with the selected carrier material and, where appropriate, the other additives such as surface-active agents. may also be prepared by micronisation, for instance in view to obtain them in the form of microspheres usually having a diameter of about 1 to 10 µm, namely for the manufacture of microcapsules for controlled or sustained release of the active ingredients. Additional ingredients may be included in order to control the duration of action of the active ingredient in the composition. Control release compositions may thus be achieved by selecting appropriate polymer carriers such as for example polyesters, polyamino acids, polyvinyl pyrrolidone, ethylene-vinyl acetate copolymers, methylcellulose, carboxymethylcellulose, protamine sulfate and the like. The rate of drug release and duration of action may also be controlled by incorporating the active ingredient into particles, e.g. microcapsules, of a polymeric substance such as hydrogels, polylactic acid, hydroxymethylcellulose, polymethyl methacrylate and the other above-described polymers. Such methods include colloid drug delivery systems like liposomes, microspheres, microemulsions, nanoparticles; nanocapsules and so on. Depending on the route of administration, the pharmaceutical composition may require protective coatings.

The pharmaceutical compositions of the invention may be administered by any route well known in the art, i.e. orally, intranasally, subcutaneously, intramuscularly, intradermally, intravenously, intra-arterially, parenterally or by catheterization.

Over-expression of sense NuSAP promotes cell proliferation and in particular promotes cell proliferation via DNA replication. Therefore the above-disclosed eukaryotic polynucleotides encoding for NuSAP, including portions thereof, may usefully be administered to a mammal in order to prevent or treat a disorder associated with decreased cell proliferation.

The NuSAP protein and the *nusap* gene can be used as a diagnostic tool in diseases were the level of DNA synthesis is disturbed, such as specific types of cancer, or in diseases that are caused by an impaired recombination. Differences in NuSAP concentration and/or amounts can be detected at the protein level by Western blots, or at the mRNA level by Northern blots or quantitative RT-PCR. As can be seen in one of the following examples, the elevated expression level of *nusap* differs depending from the cancer being considered. At the genomic level, mutations, deletions or translocations of NuSAP can be detected by hybridisation with NuSAP oligo- or polynucleotide probes or by detection procedures in which a PCR with nusap primers is performed.

NuSAP also relates to diseases caused by genetic defects of structural nuclear proteins. The following examples show that NuSAP is a constituent of a fibrous structure in the nucleus, the nuclear matrix. Diseases considered here are namely the Emery-Dreifuss muscular dystrophy caused by deletions or mutations in the *Emerin* gene or the *lamin* A gene (Bione et al. in Nature Genet. (1994) 8:323-7; Bonne et al. in Nature Genet. (1999) 21:285-8) and the dilated cardiomyopathy and lipodystrophy being caused by mutations in the *lamin* A gene (Cao et al. in Hum. Molec. Genet. (2000) 9: 109-112; Fatkin et al. in New Eng. J. Med. (1999) 341: 1715-1724).

The examples in this application demonstrate the tissue specific expression of *nusap*. *Nusap* gene expression is for instance strong in meiotic cells of the reproductive organs. Therefore, the disclosed polynucleotides encoding for NuSAP, a vector containing these sequences, NuSAP or a portion thereof may be usefully administered to a mammal in order to prevent or treat a reproductive disorder. *Nusap* gene expression is further strong in mitogenic cells of the immune system and in bone cells. Therefore, the disclosed polynucleotides encoding NuSAP, the NuSAP protein or a portion thereof may be usefully administered to a mammal in order to prevent or treat a disorder associated with the immune system or a disorder related to bone, in particular to bone growth.

The examples show the anti-proliferative effect of 1α,25-dihydroxyvitamin D3, through downregulation of *nusap* expression. 1α,25-dihydroxyvitamin D3 may thus be used to modulate *nusap* gene expression, controlling thereby or interfering with cell regulatory processes like DNA replication, DNA repair and recombination. Therefore, a therapeutically effective amount of vitamin D3 may be administered to a mammal, more preferably a human being, in order to prevent or treat a disorder associated with cell hyperproliferation.

Any of the diagnostic and therapeutic methods described herein-above may be applied to any mammal in need of such therapy or diagnosis, for example dogs, cats, cows, horses, rabbits, monkeys, and most preferably, humans.

As previously indicated, NuSAP plays a role in DNA replication and recombination. Involvement in DNA replication is suggested by colocalization to sites of bromodeoxyuridine (hereinafter referred as BrDU) incorporation (DNA replication foci) and by a correlation with *histone H4* expression (marker linked to DNA replication); upregulation of *nusap* expression during the replicative phase of the cell cycle (S-phase); restriction of *nusap* expression to actively dividing mitogenic or meiotic cells. Another biological function of NuSAP in DNA recombination derives from the fact that tissue-specific *nusap* expression does not always follow *histone H4* expression. In the adult testis, high expression was for instance confined to the primary spermatocytes in the late pachytene stage of meiosis, during which recombination is completed. In lymphoid tissues recombination occurs during immunoglobulin and T cell receptor gene arrangements and /or immunoglobulin class switching. Therefore, the disclosed polynucleotides encoding for NuSAP, a vector containing these sequences, the NuSAP protein or a portion thereof can be used in recombination and DNA replication technology, for instance for gene transfer and/or ectopic expression.

Various assays, well known in the art, may be used to detect disorders associated with *nusap* expression. Diagnostic tools may comprise antibodies raised against the NuSAP protein in its broad meaning. Such antibodies may be used for the diagnosis of conditions or diseases characterized by expression of NuSAP, or in assays to monitor patients being treated with NuSAP or its encoding polynucleotides. Monitoring protocols include for instance ELISA, radio-immune assays and fluorescence-activated cell-sorter analysis (hereinafter referred as FACS) and are well known in the art.

Polynucleotides encoding NuSAP or a portion thereof may also be used for diagnostic purposes. The polynucleotides which may be used include oligonucleotide sequences; antisense RNA and DNA molecules. They may be used to detect and quantify gene expression in biopsied tissues in which expression of NuSAP may be correlated with a disease such as above defined. The diagnostic assay may be designed and used to distinguish between the absence, the presence and the excessive expression of NuSAP, and to monitor regulation of NuSAP levels during therapeutic intervention.

In one aspect, hybridization with PCR probes which are capable of detecting polynucleotide sequences, including genomic sequences, encoding NuSAP or closely related molecules, may be used to identify polynucleotide sequences encoding NuSAP. The specificity of the probe, whether it is made from a highly specific region, e.g. 10 unique nucleotides in the 5' regulatory region, or a less specific region, e.g., especially in the 3' coding region, and the stringency of the hybridization or amplification will determine whether the probe identifies only naturally occurring sequences encoding NuSAP, alleles, or related sequences. Probes used for the detection of related sequences, should preferably contain at least about 50% of the nucleotides from any of the NuSAP encoding sequences. Means for producing specific hybridization probes and the appropriate vectors are known in the art. Hybridization probes may be labelled by a variety of well known reporter groups.

Such diagnostic tools may be used to detect any disorder associated with hyperproliferation such as cancer, a reproductive disorder or a disorder associated the bone, or may be used in the prevention and/or treatment of immune pathologies. In a particular aspect, the polynucleotides encoding NuSAP may be useful in assays that detect activation or induction of various cancers. Such assays may also used to evaluate the efficacy of a particular therapeutic treatment of the diseases enumerated herein above. In order to provide a basis for the diagnosis of disease associated with expression of NuSAP, a normal or standard profile for expression in the respective tissues is established. Once disease is established and a treatment protocol is initiated, assays may be repeated on a regular basis to evaluate progression. The results obtained may be used to show the efficacy or inefficacy of the treatment over a period ranging from several days to months.

With respect to cancer, the presence of a relatively high amount of transcript of NuSAP in a biopsied tissue from an individual may indicate a predisposition of said individual for the development of the disease, or may provide a means for detecting the disease prior to the appearance of actual symptoms. A more definitive diagnosis of this type may allow health professionals to employ preventive measures or aggressive treatment earlier thereby preventing the development of further progression of the cancer.

Additional diagnostic uses for oligonucleotides designed from the sequences encoding NuSAP involve the use of PCR. Such oligomers may be chemically synthesized, generated enzymatically, or produced from a recombinant source. Oligomers will preferably consist of two nucleotide sequences, one with sense and another with antisense orientation, employed under optimized conditions for identification of a specific gene or condition, as well known to those skilled in the art.

Methods which may also be used to quantify the expression of NuSAP include radiolabeling, fluorescent or immunolabeling of nucleotides, coamplification of a control polynucleotide , and standard curves on which the experimental results are interpolated, such as disclosed for instance by Melby et al. in J. Immunol. Methods (1993) 159:235-244 and Duplaa et al. in Anal. Biochem. (1993) 229-236.
Also, the polynucleotide sequences which encode NuSAP may be used to generate hybridization probes which are useful fro mapping the naturally occurring genomic sequence. The sequences may be mapped to a particular chromosome or to a specific region of the chromosome using well known techniques such as, but not restricted to fluorescence in situ hybridization (hereinafter referred as FISH) or FACS.

The following examples are only for the purpose of describing particular embodiments of this invention and are not intended to limit its scope, which is only defined by the appending claims. This invention is not limited to the particular methodologies, protocols, cell lines, vectors, and reagents described in the following examples as these may vary according to the general common knowledge of the skilled person. Most of the technologies used herein-after are described in Current Protocols in Molecular Biology (1999), John Wiley & Sons, New York.

### EXAMPLES

Materials and methods used were as follows:

### Cell Culture and Treatments

All culture reagents, unless otherwise mentioned, were obtained from GIBCO BRL (Life Technologies, Grand Island, New York). Murine osteoblast-like MC3T3-E1 cells (Sudo et al. (1983). J. Cell Biol. 96:191-198) were purchased from Riken Cell Bank (H. Kodama, Japan), and maintained in aMEM supplemented with 10 % heat-inactivated FCS and penicillin-streptomycin (100 U/ml and 100 mg/ml, respectively). Sub-confluent MC3T3-E1 cell cultures are defined to be in the proliferation state, while confluent and mineralising cells have entered the differentiation stage. COS-1 and NIH3T3 cells (American Type Culture Collection, Manassas, Virginia) were cultured respectively in Dulbecco's modified eagle medium (hereinafter DME) and DME-F12, plus 10 % heat-inactivated FCS. For NIH3T3 cells, the medium was supplemented with glutamax (2mM) and glucose (4.5g/l). Cultures were grown at 37°C in a humidified atmosphere containing 5 % CO₂ in air.

Synchronized MC3T3-E1 cells were obtained by a double thymidine block (Stein, et al. (1990) in "Cell Growth and Division, a practical approach". R. Baserga, editor. IRL Press at Oxford University Press, Oxford. 133-137; Ryoo et al. (1997) J. Cell. Biochem. 64:106-116). Cells were exposed twice to 2mM thymidine (Calbiochem-Novabiochem Corp., La Jolla, California) and were harvested at varying time points after release from the growth arrest.

Growth arrest of MC3T3-E1 cells by serum starvation was accomplished by changing complete medium (10% FCS) to medium containing 0.1 % FCS in subconfluent cultures. The quiescent cells were released from growth arrest by adding complete medium (10 % FCS), whereafter cells were harvested at the indicated time points. Growth arrest was also induced by treating subconfluent MC3T3-E1 cultures with 1mM hydroxyurea (Calbiochem-Novabiochem Corp).

To obtain mitotic COS-1 cell populations, 1mM hydroxyurea was added to the culture for 18 h, whereafter cells were washed and released in growth medium for 7.5 h. Nocodazole was obtained from Calbiochem-Novabiochem Corp., taxol from Sigma Chemicals Co. (St Louis, Missouri), and aphidicolin from Roche Molecular Biochemicals (Mannheim, Germany).

1α,25(OH)₂D₃ is commercially available from Hoffman-La Roche Inc., Nutley, New Jersey).

### Differential Display and RACE

Differential display was performed essentially as previously described (Liang et al. (1993) Nucleic Acids Res. 21:3269-3275). Briefly, total cellular RNA was extracted from cell cultures using TriZol LS (GIBCO BRL) and was treated with RNase free DNase I (Roche Molecular Biochemicals) to remove any residual DNA contamination. After phenol/chloroform extraction and ethanol precipitation, RNA was dissolved in H₂O and was reverse-transcribed using Superscript II (GIBCO BRL) with the following anchor primer set: 5'-T₁₂MG-3' where M represents A, C or G. The resultant cDNAs were amplified by PCR in the presence of [³³P]dATP (Amersham Pharmacia Biotech., Uppsala, Sweden) using 5'-T₁₂MG-3' and the following arbitrary decamer: 5'-ACGGTCATAG-3'. PCR products were separated on a 6 % polyacrylamide/urea sequencing gel; the gel was dried and the products were visualized by autoradiography. Bands exhibiting differential expression were excised from the dried gel, and DNA in the gel slice was eluted and reamplified using the same set of primers. The PCR products were ligated into the cloning vector pGEMTEasy (Promega Corp., Madison, Wisconsin). Screening of positive clones was carried out as previously described (Shoham et al. (1996) Biotechniques 20:182-184). Amplitaq (Perkin-Elmer Biosystems, Foster City, California) was used in all PCR steps.

The 3'-primers, that were used in the 5'-RACE kit (GIBCO BRL) were designed based on the 3'-untranslated region (UTR) sequence obtained from the differential display fragment. NuSAP mRNA was reverse-transcribed using the following primer: 5'-CAGATGGCAAATACATG-3'. PCR amplification of the resultant cDNA was performed with the following gene specific 3'-primer: 5'-CAGAACAAGGCTCTTATAAGC-3'. The PCR product was ligated into the cloning vector pGEMTEasy.

### DNA Sequence Analysis

Plasmid DNA was purified using a Nucleobond Plasmid Midikit (CLONTECH Labs, Palo Alto, California), and the sequence of all cloned fragments was determined via the dideoxynucleotide chain termination method using a fluorescent cycle sequencing kit and the ALF Express DNA sequencer from Amersham Pharmacia Biotech. The nucleic acid and deduced protein sequence were compared with known sequences in the databanks using the BLAST algorithms (Altschul (1997) Nucleic Acids Res. 25:3389-3402). The GCG software package (Genetics Computer Group, Inc., Madison, Wisconsin) was used for all sequence assembly and analysis.

### Northern Analysis

Total cellular RNA was extracted from cell cultures using TriZol LS. Foetal mice and soft tissues from adult mice (C57BL/6; 8 wk old) were directly homogenized in TriZol (GIBCO BRL), while femurs and tibias were ground to a fine powder prior to homogenization, followed by total cellular RNA extraction. 18 µg of denatured RNA was applied per lane, separated by electrophoresis in 1 % agarose/formaldehyde gels and transferred to positively charged nylon membranes (ICN Pharmaceuticals Inc., Costa Mesa, CA) as previously described (Ausubel (1999) Current Protocols in Molecular Biology, John Wiley & Sons, New York. 4.9.1-4.9.13). Membranes were prehybridized and then hybridized for 18 hours at 42° C with cDNA probes, labeled with [³²P] dCTP by random priming (Amersham Pharmacia Biotech.), in 50% formamide-containing solutions. The membranes were then washed and subjected to autoradiography at -70° C. To assess equal loading, blots were rehybridized with a radiolabelled glyceraldehyde 3-phosphate dehydrogenase (GAPDH), β-actin or an 18 S ribosomal probe. The following cDNA probes were used: human histone H4, pFO002 (Pauli et al. (1989) J. Cell Physiol. 139:320-328) mouse osteocalcin, p923 (Celeste et al. (1986) EMBO J. 5:1885-1890) and a 0.8 kb NuSAP BglII/AspI restriction fragment.

### In Vitro Transcription and Translation

The coding region of NuSAP was amplified using *pyrococcus furiosus* (hereinafter referred as Pfu) polymerase (Stratagene, La Jolla, California) with the following specific primers, 5'-CGAGATTGCAGAACGCGATGAC-3' and 5'-TTAGAGTAAAGGAACAGACAGGG-3'. The PCR fragment was blunt-end ligated into the pCRScript plasmid (Stratagene) and sequenced to verify NuSAP sequence and proper reading frame. The plasmid DNA was transcribed and translated using the TNT T7 reticulocyte lysate system (Promega Corp.) with translation grade L-[³⁵S]cysteine (1000 Ci/mmol; Amersham Pharmacia Biotech). Translation products were separated by SDS-polyacrylamide gel electrophoresis and processed for fluorography. Luciferase DNA was used as a positive control. Treatment with calf intestine alkaline phosphatase (GIBCO BRL) was for 30 minutes at 37°C.

### Construction of NuSAP Vectors

The NuSAP sense expression vector was designed to contain the coding sequences of NuSAP fused upstream to the Myc- and His-tag of the pcDNA3.1 (-)/Myc-His B plasmid (Invitrogen Corp., Carlsbad, California). The following specific primers incorporating a 5'-NotI and a 3'-BamHI site were used in the amplification reaction: 5'-TAAGGATCCAAGTTCACACCCAGGTTTCTTCG-3' and 5'-ATAAGAATGCGGCCGCCATGGCCGTCCCCTCTGCAGAGGAGCTG-3'. The digested PCR fragment was inserted into the complementary sites of the pcDNA3.1(-)/Myc-His B plasmid.

The NuSAP antisense vector was engineered by cloning the NotI-BamHI insert from the pCRScript vector used in the transcription and translation experiments (see supra) into the corresponding sites of the pcDNA3.1(-)/Myc-His A plasmid.
The NuSAP-GFP fusion vector was designed to contain the coding sequences of NuSAP fused upstream to the green fluorescent protein (GFP) sequence of the pEGFP-N1 plasmid (CLONTECH). The following specific primers incorporating a 5'-SacI and a 3'-BamHI site were used in the PCR reaction: 5'-TAAGGATCCAAGTTCACACCCAGGTTTCTTCG-3', and 5'-CTTGAGCTCTACCCGAGATTGCAGAACGCG-3'. The digested PCR fragment was inserted into the complementary sites of the pEGFP-N1 plasmid.

The myc- and flag-tagged NuSAP vectors were constructed after removing the GFP-tag by digesting with BamHI and NotI, and inserting a myc and flag adapter duplex containing these restriction sites. The following oligonucleotides were used: 5'-GATCTAGAACAAAAACTCATCTCAGAAGAGGATCTGTGA-3' and 5'-GGCCTCACAGATCCTCTTCTGAGATGAGTTTTTGTTCTA-3' for the myc-tagged vector and 5'-GATCTAGACTACAAGGACGACGACGACAAGTGA-3' and 5'-GGCCTCACTTGTCGTCGTCGTCCTTGTAGTCTA- 3' for the flag-tagged vector.

In all cases, Pfu polymerase (Stratagene) was used in the amplification reaction. All plasmids were sequenced to verify junctions and to ensure the proper NuSAP sequence.

HuNuSAP cDNA containing the protein coding sequence was cloned via PCR using cDNA derived from RNA isolated from bone tissue of an adult human female. Cloned in pcDNA3.1(-)Myc-His using the following primers:
1) 5'-CGCGGATCCTTCAGCCAAAATGAGGCCCCTTC-3'
2) 5'-ATAAGAATGCGGCCGCCATGGTCATCCCCTCTCTAGAGGAGC-3'

### [³H]-Thymidine Incorporation

COS-1 and NIH3T3 cells were plated in flat bottomed 96 well plates (Nalge Nunc International, Rochester, New York), respectively at 2000 and 750 cells per well. After one day, cells were transfected using Fugene 6 (Roche Molecular Biochemicals) according to the manufacturer's instructions with the NuSAP-sense and -antisense expression vectors and pcDNA3.1(-)/Myc-His B plasmid as a control. For each condition there were at least 6 replicates and experiments were repeated at least 3 times. Plasmid DNA derived from independent DNA preparations was also used.

[³H]-thymidine incorporation was performed 48 hours post transfection. 1 µCi [³H]-thymidine (2 Ci/mmol; ICN Pharmaceuticals Inc.) was added to each well for 4 hours at 37°C in 5% CO₂, whereafter the cells were harvested using a micro cell harvester (Filtermate Universal Harvester, Packard Instrument Co., Meriden, Connecticut) on glass filter membranes (Unifilter, Packard Instrument Co.). After drying and addition of Microscint-O scintillation liquid (Packard Instrument Co.), microplates were counted in a TopCount Microplate Scintillation Counter (Packard Instrument Co.).

### Antibodies

The following antibodies were used to detect epitope tagged NuSAP: for immunoprecipitation analysis, a rabbit polyclonal Ab (pAb) to GFP (Molecular Probes, Eugene, OR) was used and for immunofluorescence analysis, a rabbit pAb against c-myc (Santa Cruz Biotechnology, Santa Cruz, California), and a mouse mAb to flag (M2) (Sigma Chemicals Co.) were used. Goat pAb against lamin A/ C were obtained from Santa Cruz Inc. (Santa Cruz, California). The mouse monoclonal anti-α-tubulin antibody (Blose (1984) J. Cell Biol. 98:847-858) was purchased from Amersham Pharmacia Biotech. The mouse monoclonal anti-C23 (nucleolin) antibody was purchased from Santa Cruz Biotechnology, Inc.. The secondary Texas Red-labeled goat anti-mouse antibody was purchased from Molecular Probes, Inc. BSA (fraction V) was purchased from Sigma Chemicals Co. The mouse monoclonal anti-His (C-terminal) antibody was purchased from Invitrogen Corporation. A 5-Bromo-2'-deoxy-uridine (BrdU) labeling and detection kit (Roche Molecular Biochemicals) was used according to the manufacturer's instructions to detect BrdU incorporated into cellular DNA. Actin was detected using Texas Red-X-Phalloidin (Molecular Probes).

### Immune fluorescence microscopy

COS-1 cells were plated in Lab-Tek II chamber slides (Nalge Nunc International) and transfected with the NuSAP-GFP fusion vector using Fugene 6 according to the manufacturer's instructions. Immunofluorescence analysis was performed 24 hours after transfection. Cells were briefly washed in phosphate buffered saline (PBS), fixed in methanol/acetone (1/1) and were air dried. Cells were then washed 3 times in 0.1% Tween 20/PBS, and incubated for 20 minutes at room temperature with 0.5% bovine serum albumin (BSA)/PBS, pH 7.4. Thereafter cells were incubated with primary antibody at 2 µg/ml in 0.5% BSA/PBS for 1 hour at room temperature, washed 4 times in 0.5 % BSA/PBS, and incubated with the secondary antibody at 6 µg/ml in 0.5 % BSA/PBS for 1 hour at room temperature. Cells were then washed 4 times in PBS prior to post-fixation in 3.7 % paraformaldehyde/PBS for 30 minutes. The chromosomes were stained by incubating the cells for 5 minutes in PBS containing 2µg/ml Hoechst 33258 (Sigma Chemicals Co.). Cells were mounted in DAKO fluorescent mounting medium (DAKO, Glostrup, Denmark).

Alternatively, cells were fixed and permeabilized in 0.4% glutaraldehyde/ 0.5% Triton X100/ PBS for 15 minutes at room temperature. After incubation in 0.5 mg NaBH4/ ml PBS (10 minutes), and blocking in 5%BSA/ PBS (10 minutes), cells were incubated with primary antibodies for 1 h. Cells were washed and after incubation with 50% normal serum (Dako) (10 minutes), cells were incubated with secondary antibodies conjugated to Alexa-488 or -546 dye (Molecular Probes) (1 hour) and finally mounted using Dako mounting medium. Antibodies and normal serum were diluted in 0.5% Tween20/ PBS. Analysis was done on a Nikon inverted microscope Diaphot 300 (Plan Apo 60/ 1.40 oil) connected to a Bio-Rad MRC1024 confocal microscope and images were captured by LaserSharp (version 3.2) and processed using Adobe Photoshop 5.5 (Adobe Systems Inc., San Jose California). The images showing a-tubulin localization were taken using a Leitz DM microscope and were recorded using a cooled charge-coupled device camera (Photometrics Inc., Tucson, Arizona) and IP-Lab software. The presence of the filamentous network was also confirmed using other fixation and permeabilization agents which included 0.8% paraformaldehyde/ 0.5% 7 Triton X100/PBS, methanol/ acetone 1:1 (volume/voume), and 4% paraformaldehyde. For BrdUrd labeling, cells were incubated with 10 µmol BrdUrd (15 minutes) prior to fixation, and treated with 0.07N NaOH (2.5 min) prior to the BSA block.

### Nuclear Extraction for Microscopy

24 hours after transfection, COS-1 cells were briefly washed in PBS and subjected to permeabilization and cell extraction as described (Fey et al. (1984) J. Cell Biol. 98:1973-1984). Cells were extracted with CSK buffer (0.5 % Triton X-100 in 10 mM Pipes, pH 7.0, 100 mM NaCl, 300 mM sucrose, 3 mM MgCl₂, and 1 mM EGTA (Ethylene glycol-bis(2-aminoethyl)-N,N,N',N'-tetraacetic acid) containing 1 mM phenylmethylsulfonyl fluoride (PMSF) twice for 15 minutes each at 4°C . Thereafter, RNA or DNA were digested twice with 100 µg/ml RNase A and 100 µg/ml DNase I (Roche Molecular Biochemicals) respectively for 30 minutes each in digestion buffer (CSK buffer with 50 mM NaCl) at 30°C. Digested chromatin was extracted at 4°C with 250 mM ammonium sulfate in the digestion buffer for 10 minutes and again for 5 minutes. Cells were fixed for immunofluorescence microscopy as described above.

### Immunoprecipitation and Western Blot Analysis.

Cell extracts were prepared 24 hours after transfecting COS-1 cells with the NuSAP-GFP expression vector or the empty GFP vector, as a control. After washing in ice-cold PBS, cells were harvested by scraping in PBS. The cells were pelleted and resuspended in ice- cold lysis buffer (50mM Tris HCl (pH7.4), 0.5% Triton X100, 0.1% NP-40, 150 mM NaCl, 1 mM EDTA, 0.25% gelatine, 1 mM PMSF, 10 µg/ ml aprotinin, 10 µg/ ml leupeptin, and 1 µg/ ml antipain). Cells were homogenized by passing the mixture several times through a G26-needle and subsequently centrifuged at 10000 X g (15 minutes at 4°C). Extracts were then precleared (40 minutes at 4°C) using control serum and protein-A or -G agarose beads (Santa Cruz), and subsequently incubated with specific antibodies and beads (1 hour at 4°C) under rotation. Beads were pelleted, washed three times in lysis buffer and the precipitated protein complexes were eluted by boiling in SDS sample buffer. Proteins were seperated by sodium dodecyl sulfate polyacrylamide gel electrophoresis (SDS-PAGE), and were transferred to a nitrocellulose membrane. Membranes were probed with the relevant primary and secondary antibodies (Dako) and developed by enhanced chemiluminescence (ECL system, New England Nuclear) according to the manufacturers instructions.

### Fluorescence-Activated Cell Sorter (FACS) Analysis.

Sub-confluent COS 1 cells were collected by trypsinization 24 or 48 hours after transfection with the NuSAP-GFP or empty GFP expression vectors. Cells were first fixed for 45 minutes at 4 °C in CellFIX solution (Becton Dickinson) for the preservation of GFP fluorescence. Subsequently, cells were fixed for 2 hours at 4°C in 70% ethanol as required for DNA staining. After washing twice in PBS, cells were resuspended in a solution containing 25 µg/ 8 ml propidium iodide (Calbiochem), 100 µg/ ml RNase A (Sigma) and 0.5% Triton X100 and incubated for 45 minutes at room temperature. Cells were immediately analyzed for DNA content by flow cytometry (FACScan, Becton Dickinson). Analysis of DNA content was restricted to GFP-expressing cells, and was repeated at least three times using independently transfected cell populations. Different plasmid DNA preparations were also tested.

### In Situ Hybridization

Digoxigenin-11-UTP-labelled single-strand RNA probes were prepared using a DIG RNA labeling kit (Roche Molecular Biochemicals) according to the manufacturer's instructions.

The NuSAP cDNA fragment was used to generate antisense and sense probes, which were reduced to an average size of 100 bases by limited alkaline hydrolysis.

Embryo's and organs of adult mice were fixed in 2 % paraformaldehyde in PBS by perfusion, incubated at 4° C overnight and then washed, dehydrated and embedded in paraffin wax (Hoang et al. (1998) Dev. Dyn. 212:364-372). Sections of 8 µm were cut (Leica RM 2155, Leica Microsystems Inc., Deerfield, IL), transferred to positively charged Optiplus slides (Biogenex, Sam Ramon, California) and dried overnight at 37°C. After deparaffinization, sections were treated with 10 µg/ml of proteinase K (Roche Molecular Biochemicals) for 10 minutes at room temperature and thereafter fixed in 4 % paraformaldehyde in PBS for 10 minutes. Next, the slides were washed with PBS and treated with acetic anhydride (0.25 %). Hybridization was performed at 70°C in 50 % formamide, and washes were carried out at a stringency of 0.2X SSC containing 50 % formamide at 70°C. A DIG nucleic acid detection kit (Roche Molecular Biochemicals) was used according to the manufacturer's instructions to detect signals.

### EXAMPLE 1 - Identification and Isolation of the Mouse nusap cDNA

Treatment of mouse osteoblastic MC3T3-E1 cells with 1α,25(OH)₂D₃ inhibited the proliferation of these cells as measured by [³H]-thymidine incorporation (Fig. 1). Significant effects were already observed after 24 hours and this inhibition became more pronounced with increasing time. To obtain more insight in this anti-proliferative effect, we used mRNA differential display analysis to compare gene expression after treatment with 1α,25(OH)₂D₃ (10⁻⁸ M) or vehicle for 24 hours. A cDNA fragment that showed reduced levels after 1α,25(OH)₂D₃ treatment was cloned. Similarly, the gene level of this particular cDNA clone is also lower in a differential display of undifferentiated cells (subconfluent cell culture) compared with differentiated cells (confluent, mineralized cell culture). Northern analysis using this cDNA fragment as a probe confirmed the inhibition of *nusap* gene expression after treatment and also revealed the presence of three transcripts: one major 2.4 kb transcript and two minor transcripts of 1.7 kb and 4.5kb (Fig. 2). Comparing *nusap* gene expression with molecular markers for growth *(histone H4*) and differentiation *(osteocalcin)* in long term osteoblast cultures (reviewed in Stein, et al. (1996) Physiol. Rev. 76:593-629), showed a clear association of *nusap* gene expression with proliferation (Fig. 3).

The full-length sequence of the major 2.4 kb transcript was obtained via 5'RACE using gene specific primers based on the differential display cDNA fragment. The entire cDNA was sequenced and the single long open reading frame was analyzed (fig. 4). Both mouse and human sequences were cloned and BLASTP (Altschul et al. (1997) Nucleic Acids Res. 25:3389-3402) searches revealed that the predicted protein sequences were identical to the sequences of recently submitted, but unpublished proteins present in lymphocytes (GENBANK accession no. AAG31285 & AAG25874).

### EXAMPLE 2 - Analysis of the mouse NuSAP Protein Sequence

Translation of *nusap* cDNA is predicted to start at the first methionine positioned at nucleotide 71 and to terminate at nucleotide 1352. The first ATG lacks an ideal Kozak context (Kozak. (1991) J. Biol. Chem. 266:19867-19870) but an accessible 5'UTR (49% GC) can however compensate for the less favorable ribosome binding-site context. The 70 bp 5'UTR is followed by a 1281 bp coding region and a 785 bp 3'UTR containing two consensus polyadenylation signals (AATAAA) positioned at nucleotides 1885 and 2123 (SEQ ID NO: 1). Differential usage of these signals can clarify the minor 1.7 kb transcript (Fig. 2). The presence of in-frame termination codons in both 5'- and 3'-UTRs supports the correct assignment of the initiator methionine.

The major open reading frame (ORF) contained in the *nusap* cDNA predicts a protein of 427 amino acids with a calculated molecular weight of 48.6 kD and a predicted isoelectric point (pI) of 9.9. The highly charged protein has 141 charged residues, of which 85 are basic. The many lysine residues (K), which make up about 12% of the protein, are more abundant towards the C terminus. The protein possesses few major hydrophobic amino acid residues. A hydropathy plot, according to Kyte et al. in J. Mol. Biol. (1982) 157:105-132, did not show any apparent membrane-spanning region nor was a N-terminal signal sequence detected.

Examination of the predicted sequence also identified two types of potential nuclear localization signals (NLSs) (Dingwall et al. (1991) Trends Biochem. Sci. 16:478-481): a single stretch of four basic residues that appears several times and one bipartite nuclear localization signal (NLS) (Fig. 4).

The search for protein motifs also indicates the presence of a sequence in the aminoterminal part of NuSAP, which is highly reminescent of a SAP motif (Fig. 4). The SAP motif is a DNA binding motif involved in chromosomal organization (Aravind et al., cited *supra*). The putative SAP motif of NuSAP contains many spaced leucine residues which comply with the consensus sequence. It has a similar predicted secondary structure (via PredictProtein) where two alpha-helices are formed separated by a turn , and resides at the N-terminus of the protein, a feature that is observed in many other proteins containing this motif (figure 4). The 'invariant' glycine residue of the SAP motif, which is found between the two helices, is not conserved and is replaced by a hydrophobic residue (leucine or methionine) probably resulting in a variant turn.

The NuSAP protein sequence contains multiple consensus protein phosphorylation sites. Several putative casein kinase II, protein kinase C (PKC) and one cAMP- and cGMP-dependent protein kinase phosphorylation sites were identified in NuSAP using the PROSITE algorithm (Hofmann et al. in Nucleic Acids Res. (1999) 27:215-9). Based on studies of cdc2 kinase phosphorylation sites in the major nucleolar protein nucleolin, a consensus sequence for mitotically active cdc2 kinase (X-T/S-P-X-K/R) was developed (Peter et al. (1990) Cell. 60:791-801). The NuSAP sequence possesses three of these consensus cdc2 kinase phosphorylation sites, two of which are completely conserved between the mouse and human sequence. The PROSITE databank search also identified several potential N-myristoylation, one N-glycosylation site and one putative glycosaminoglycan attachment site.

The protein seems to be evolutionary conserved as there is significant sequence similarity (>60 % at the protein level) between the mouse NuSAP protein and the human homologue

### EXAMPLE 3 - Characterization of the Mouse nusap Gene Product

To confirm that the presumptive open reading frame can produce a protein of the expected molecular mass of 48.6 kD, in vitro transcription and translation was performed using *nusap* cDNA containing the predicted ORF. A protein of approximately 55 kD was detected on SDS PAGE. The difference observed between the apparent and predicted molecular mass can be due to the multiple consensus phosphorylation sites present in the protein sequence. To test this hypothesis we treated the in vitro transcription and translation product with alkaline phosphatase for 30 minutes and observed a small band shift, indicating a change in molecular mass by the removal of phosphate groups. The difference between predicted and apparent molecular mass can in part be ascribed to this post-translational modification. Another plausible explanation is that, due to the highly charged nature of the protein, it exhibits an apparent molecular mass on SDS PAGE significantly higher than predicted from its amino acid sequence as has been described for other proteins (Mortillaro et al. J. Biol. Chem. 273:8183-8192). Alternatively, other post-translational modifications can account for the difference in molecular mass.

### EXAMPLE 4 - Proliferation- and Cell Cycle-Specific Expression of the Mouse nusap Gene in MC3T3-E1 Cells

The expression of the *nusap* gene was shown to correlate with the proliferation phase during osteoblast development (Fig. 3). To further characterize this proliferation-specific expression of *nusap* growth arrest was induced in MC3T3-E1 cells by serum starvation and RNA was extracted at the indicated time points (Fig. 5). A clear reduction in *nusap* RNA levels was observed 8 hours after withdrawal of serum. When complete growth medium was added to the quiescent MC3T3-E1 cells, allowing them to proliferate, near maximal levels of *nusap* gene expression were reached within 24 hours. A comparable expression pattern was observed for the *histone H4* gene. When growth arrest of cells was induced by hydroxyurea, a ribonucleotide reductase inhibitor (Timson, (1975) Mutat. Res. 32:115-132), both *nusap* and *histone H4* RNA levels quickly declined (Fig. 6). Within 45 minutes of treatment *histone H4* RNA levels became undetectable, and *nusap* gene expression was markedly down-regulated. Knowing that *histone H4* gene expression is directly coupled to DNA replication these data suggest that the expression of *nusap* is linked to DNA replication, but not directly coupled to it.

Based on the association with cell proliferation, we further examined whether *nusap* RNA levels were altered during progression of the cell cycle. A double thymidine block was used to synchronize MC3T3-E1 cells at the G1/ S phase boundary (Fig. 7). The different cell cycle phases were assigned by determining the message levels of *histone H4,* a S phase marker, as has been described by Ryoo et al. in (1997) J. Cell. Biochem. 64:106-116) using the same cell line. Northern analysis showed high RNA levels of *nusap* 4 to 6 hours after release from growth arrest. Comparing *nusap* RNA levels with those of *histone H4* revealed that *nusap* gene expression was up-regulated during the S and G2 phase of the cell cycle (Fig. 7). After release for 8 to 10 hours (M/ G1 phases) *nusap* message levels declined, to increase again in the subsequent S phase. These data indicate that *nusap* gene expression is regulated during the cell cycle.

### EXAMPLE 5 - Mouse nusap Expression is Tissue-Specific

To further characterize the *nusap* gene and its biological activity, we examined the expression pattern of *nusap* in adult mice tissues. Northern analysis of total (Fig. 8) and poly(A)+ RNA extracted from adult mice tissues (C57BL/6 strain) showed that the gene was expressed in organs of the immune system (thymus, spleen, lymph node), organs of the reproductive system (testis, ovary, uterus), lung, small intestine, and bone. The highest levels were detected in the thymus, testis and bone. Interestingly, despite a high expression in the testis, the 4.5 kb minor transcript was not detectable. *Histone H4* gene expression was also analyzed as a marker of the proliferation state of the organs (Fig. 8). Comparing the levels of expression of the two genes, it is evidenced that in some organs, like the thymus, they are well correlated. There is however no strict correlation in every organ examined: the central nervous system and kidney show higher RNA levels for *histone H4* than for *nusap,* while in the testis, the opposite is observed.

Northern analysis of total RNA extracted from different developmental stages of whole mouse embryo's (C57BL/6) showed that the *nusap* gene was expressed at higher levels in younger embryos (Fig. 8). The level of expression of the *nusap* gene declined with increasing days post-coitum (dpc): 10 dpc > 13 dpc > 16 dpc > 19 dpc. The *histone H4* gene showed a comparable expression pattern (Fig. 8b). Analysis of expressed sequence tag (EST) entries at GenBank using the BLASTN algorithm (Altschul et al. (1997) Nucleic Acids Res. 25:3389-3402.) revealed that *nusap* is expressed as early as the 2-cell stadium in mice (accession No. AI505293).

To investigate whether *nusap* gene expression was cell-specific, we analyzed tissues from embryo's and adult mice (C57BL/6) by in situ hybridization (Fig. 9). Interestingly, in the adult testis (Fig. 9a) the mitotically active spermatogonia within the seminiferous tubule showed little or no expression of the gene. High expression was confined to the primary spermatocytes undergoing meiosis. By staging the seminiferous tubule, high expression of the *nusap* gene is predicted to correlate with the late pachytene stage of prophase during meiosis. The absence of expression of *nusap* in some tubules can be explained by the fact that not all tubules undergo the same stage of development. Little or no expression was observed in the interstitial cells of the seminiferous tubules, in the Sertoli cells, in the seminiferous epithelium, and in cells in spermiogenesis.

In tissue sections of adult ovary there was little or no *nusap* expression in the proliferating follicular epithelial cells and in the granulosa cells. High expression was confined to the oocytes (Fig. 9b). The thecal cells surrounding the follicular epithelial cells were also negative for *nusap* expression.

In the adult mouse thymus (Fig. 9c), high expression of *nusap* was localized to the sub-capsular outer cortex region containing actively proliferating immature T-stem cells. Little or no nusap-positive staining was observed in the cortex, where cells are probably in a resting stage, and in the medulla, where most cells are differentiated.

In the small intestine of a 19 dpc mouse embryo (Fig. 9d), the absorptive epithelial cells of the villi showed little or no staining in contrast to the mitotically active cells in the crypts that showed strong staining for *nusap* expression. The cells of the lamina propria, the supporting layer underlying the intestinal epithelium showed little or no *nusap* expression. In all tissue sections examined, no staining was observed when the sense probe was used.

These data show that *nusap* gene expression is tissue-specific and restricted in reproductive organs to cells in meiosis, and in other tissues to mitotically active cells.

### EXAMPLE 6 - NuSAP is overexpressed in Different Types of Cancer.

A 200 bp fragment of human nusap cDNA was labeled with [32P] labeled random primers. This probe was used to hybridize a panel of cDNA's derived from normal and cancer tissues according to the manufacturers instructions (matched tumor/normal expression array, (Clontech, Palo Alto). Quatitative detection was performed on a phosphorimager.

*nusap* was hybridised to a membrane on which cDNA, synthesised from 68 tumors and coresponding normal tissue from the same individual, have been immobilised (Clontech matched tumor/normal expression array). This blot contains cDNA pools of tumors from kidney, breast , prostate, uterus, ovary, cervix, colon, lung, stomach, rectum, and small intestine. As shown in figure 10, in 8 of the 11 tissues represented in this blot, a clear overexpression of nusap is seen in tumors compared to normal tissue. As can be seen from this figure, the ratio of nusap expression in tumors compared to normal tissue differs from tumor to tumor. this property can be used to diagnose and discriminate different types of cancer in one organ.

Figure 10 shows the elevated expression levels of nusap in cDNA pools of human tumor tissue (designated as T) as compared to the expression of nusap incDNA pools obtained from the same tissue from healthy individuals (designated as N). Quantitative detecture of nusap is detected by exposure on a phosphorimager.

### EXAMPLE 7 - NuSAP is a nuclear protein with a discrete distribution.

Immunofluorescence microscopy was used to analyze the subcellular distribution of mouse NuSAP in MC3T3E1 and COS1 cells. An expression vector producing full length mouse NuSAP fused at its C-terminus to a GFP-tag was constructed and used in transient transfection experiments. Most of the observations were confirmed using myc- and flag-tagged NuSAP expression vectors.

As expected from the presence of a potential nuclear localization signal, fluorescence from GFP-tagged NuSAP was restricted to the nucleus (Fig. 11A and D), as evidenced by immunostaining for lamin A/C (Fig. 11D). Within the nucleus, GFP-tagged NuSAP was predominantly observed within the nucleolus as demonstrated by nucleolin colocalization (Fig. 11A-C). Compared to nucleolin, that especially resides at the fibrillar component (Ginisty et al. (1999) J. Cell Sci. 112 761-772), GFP-tagged NuSAP was found to localize both to the fibrillar component and the fibrillar center.

To investigate whether NuSAP and nucleolin interacted directly with each other, co-immunoprecipitation experiments were carried out on extracts from transfected COS 1 cells (Fig. 11E). Immunoprecipitation with antibodies directed to the GFP-tag not only resulted in the precipitation of the NuSAP-GFP fusion protein (80 kDa) but also clearly demonstrated the presence of nucleolin (100 kDa). Reversely, when antibodies directed to nucleolin were used, the immunoprecipitate contained not only nucleolin but also NuSAP-GFP fusion protein.

Detailed confocal laser scanning microscopy revealed that fluorescence from GFP- (and myc-or flag-) tagged NuSAP, as opposed to nucleolin immunostaining, was in addition observed in organized structures within the nucleoplasm of transfected interphase COS1 cells. Successive horizontal plane sections of the nucleus taken at 1 µm (Fig. 11F-H) z axis intervals showed the presence of a filamentous network that was most clearly visible towards the periphery of the nucleus (Fig. *4F* and G-01). The filaments were seen crisscrossing the nucleus, though in sections taken at the center of the nucleus (Fig. 11H) a more diffuse fluorescence pattern was observed suggesting a possible further ramification of the network at the nuclear core. The filaments were distinct from the nuclear lamins as demonstrated by immunostaining for lamins A/C and B. Also, no colocalization was observed with tubulin and actin using specific anti-tubulin antibodies and phalloidin (data not shown). These staining patterns were observed in every transfected interphase cell using different fixatives and permeabilizing agents.

In addition to its nucleolar and filamentous localization, GFP-tagged NuSAP was also observed in multiple small foci throughout the nucleoplasm. Confocal analysis of staining for NuSAP and incorporated BrdUrd, a marker for newly replicated DNA, showed that BrdUrd foci localized adjacent to NuSAP foci (Fig. 12A-C). Treatment of transfected cells with aphidicolin, a DNA synthesis inhibitor, (20 µg/ ml for several hours) resulted in loss of immunostaining for BrdUrd though fluorescence from GFP-tagged NuSAP persisted (data not shown) indicating an indirect role of the protein in DNA replication. As a likely consequence of overexpression, the small NuSAP positive foci sometimes clustered together with BrdUrd foci in very large aggregates (Fig. 12D), which at reduced illumination intensities were clearly granular.

The observations described above were similarly made using myc- and flag-tagged NuSAP. Interestingly, GFP-tagged NuSAP shows a specific intracellular localization, not only during interphase, but also during mitosis. Double staining for GFP-tagged NuSAP and a-tubulin, in cells fixed during mitosis, showed that during metaphase and anaphase, NuSAP localizes to the microtubules of the spindle apparatus and the spindle poles. NuSAP's name derives from this initial observation: NUclear Spindle-Associating Protein.

### EXAMPLE 8 - NuSAP Relocalizes to the Chromosomes and Microtubules During Mitosis

In a next step the subcellular distribution of GFP-tagged NuSAP during mitosis was investigated. During this phase of the cell cycle certain nucleolar proteins (i.e. B23 and fibrillarin) localize to the chromosome periphery, while others localize to the nucleolar organizing region (Hernandez-Verdun et al. (1994) Bioessays. 16:179-185). Synchronization of transfected COS-1 cells with hydroxyurea, confirmed that during interphase the protein is predominantly localized to the nucleolus. In addition these experiments showed that as cells enter mitosis NuSAP translocates and associates with the mitotic chromosomes (Fig. 13). During early prophase, chromatin condensation begins at the nuclear envelope (Comings et al. (1971) Exp. Cell Res. 63:471-473) and continues until the individual chromosomes are visible. During this period GFP-tagged NuSAP associates with the chromosomes and fluorescence is most intense. In metaphase, the chromosomal association disappears and NuSAP localizes to the spindle associating with the microtubules and the brightly fluorescing spindle poles (Fig. 13). This was shown via colocalization experiments with α-tubulin, a microtubule subunit (Blose et al. (1984) J. Cell Biol. 98:847-858.). This spatial organization continues through anaphase, but during telophase NuSAP, in addition to being associated with the microtubules, which is especially visible in the midbody, also relocalizes to the chromosomes.

Further evidence for an association of NuSAP with the microtubules of the mitotic spindle was obtained using both nocodazole, a microtubule depolymerizer (Jordan et al. (1992) J. Cell Sci. 102:401-416), and taxol, a microtubule stabilizer (De Brabander et al. (1981) Proc. Natl. Acad. Sci. USA. 78:5608-5612). When COS-1 cells transfected with GFP-tagged NuSAP were synchronized to obtain a population in mitosis and thereafter incubated with nocodazole (10 µM) for 1 hour NuSAP and α-tubulin redistributed throughout the cell (Fig. 14). However, when nocodazole was washed out and the mitotic spindles were allowed to re-assemble for 1 h NuSAP relocated to the microtubules and spindle poles. Furthermore, incubation of mitotic cells with taxol (5 µM for 1 hour) which stabilizes microtubules and induces mitotic asters which are devoid of centrosomes (De Brabander et al., cited *supra*) resulted in NuSAPs translocation to these structures (Fig. 14). Neither nocodazole nor taxol treatment affected the distribution of NuSAP in the nucleoli during interphase.

### EXAMPLE 9 - NuSAP-Sense and -Antisense Expression Alters DNA Synthesis

To evaluate the role of NuSAP in cell proliferation and DNA replication, COS-1 and NIH3T3 cells were transfected with nusap-sense, -antisense, and control (without an insert) expression vectors and tested for [³H]-thymidine incorporation 48 hours later (Fig. 15 and 16). COS-1 cells transfected with the *nusap* sense expression vector showed an increase of 14 % in thymidine uptake (*P* < 0.01) compared to the control. Expression of antisense *nusap* in both COS-1 and NIH3T3 cells inhibited the thymidine uptake by 7.7 *% (P* < 0.01) and 8.4 % *(P* < 0.05) respectively, compared to the control. These data show that NuSAP has a moderate positive effect on cell proliferation.

### EXAMPLE 10 - Effect of NuSAP Overexpression on the Cell Cycle

Because fluorescence from GFP-tagged NuSAP is found juxtaposed to sites of BrdUrd incorporation in transfected COS 1 cells, the question we asked was whether NuSAP overexpression affected cell cycle progression. Using FACS analysis, COS1 cells, transfected with the NuSAP-GFP expression vector or the empty GFP-expression vector (as a control), were analyzed both at 24 and 48 hours post transfection. Cells expressing the NuSAP-GFP fusion protein showed a clear increase in the S-phase population, relative to the control, that was most obvious at 48 hours post transfection (Fig. 17). Arrest in S-phase progression was accompanied by a decrease in the population of cells in the G0/ G1-phase.

### EXAMPLE 11 - Generation of a NuSAP mouse knockout.

The NuSAP targeting vector contains a 5' flanking 5.2-kb SacI-Eco restriction enzyme (RI) and a 3' flanking 4.5-kb EcoRI fragment encompassing exon 2 (containing the ATG). A 'floxed' cassette containing the neomycin phosphotransferase gene was inserted at the EcoRI site upstream of exon 2 and a third lox site was introduced downstream of exon 2 at another EcoRI site that will be inactivated. This vector was electroporated in R1 embryonic stem cells and selection was performed with G418 (200 µg/ml) and gancyclovir (2 µM) and correct targeting was be assessed by Southern analysis using an external 1.5-kb SacI probe and an internal 1-kb NcoI-PstI probe. Targeted ES cell clones were electroporated with a Cre-recombinase expression cassette and Southern analysis demonstrated correct excision of the 'floxed' neo cassette. Chimeric mice were generated by morula aggregation and bred for germline transmission. NuSAP deficient mice were obtained by crossing NuSAP*^{lox}* mice with PGK-cre mice (Lallemand, Y., Luria, V., Haffner-Krausz, R. and Lonai, P. (1998) Transgenic Res 7:105-112.), while tissue-specific inactivation of NuSAP was obtained by crossing NuSAP*^{lox}* mice with transgenic mice expressing cre tissue-specific.

### EXAMPLE 12 - generation of a transgenic mouse over-expressing NuSAP

This example provides a transgenic rodent, in particular a mouse, whose genome contains a NuSAP gene coding for SEQ ID NO:1 or its rodent homologue for the expression of the protein according to SEQ ID NO:2 or its rodent homologue, further comprising a promoter in front of the gene for modulating the expression of the protein in comparison with wild type.

The NuSAP targeting vector contains a 5' flanking 5.2-kb SacI-Eco restriction enzyme (RI) and a 3' flanking 4.5-kb EcoRI fragment encompassing exon 2 (containing the ATG) in which an strong inducible promoter (e.g. tetracycline promoter) is placed in front of the NuSAP gene. This vector is electroporated in R1 embryonic stem cells and selection was performed with G418 (200 µg/ml) and gancyclovir (2 µM) and correct targeting was assessed. Overexpression of nusap in these cells was tested prior to aggregation. Chimeric mice are generated by morula aggregation and bred for germline transmission.

Expression of nusap in these mice occurs after addition of tetracycline and can be used as a model for screening anti cancer drug compounds since a mouse overexpressing *nusap* is more susceptible to be affected by carcinogens.

The teachings of the various above examples may be summarized as follows. The present invention identifies a novel nuclear gene named *nusap* with the following characteristics: (i) the expression is tissue-specific (especially in organs of the immune and reproductive system, and in bone) (ii) expression is associated with high RNA levels in meiotic and actively proliferating cells where (iii) its expression is cell cycle-dependent and (iv) where NuSAP protein associates with the nucleolus and elaborate fibrous network in nucleus (v) altering hereby cell proliferation in a still undefined way. NuSAPs remarkable associations with different subcellular structures indicate that it is a highly dynamic protein: during the interphase of proliferating cells, NuSAP localizes predominantly to the nucleolus but is also found adjacent to sites of DNA replication; in mitotic cells, NuSAP relocates initially to the condensing chromosomes and then associates with the microtubules of the mitotic spindle to eventually reassociate with the chromosomes at the end of mitosis.

### NuSAP Has a Putative SAP Motif

Especially interesting in the light of NuSAP's proposed structural role in coordinating spatial regulation of DNA replication, is the potential existence of a SAP motif within the protein. This is a putative DNA binding motif involved in chromosomal organization (Aravind et al., cited *supra*). Assignment of the motif is based on sequence similarity, predicted secondary structure similarity, and a N-terminal localization. The presence of this motif points to a participation in additional nuclear processes, besides DNA replication. NuSAP's gene expression pattern relates to this aspect. A structural role in DNA recombination is envisaged based on its tissue-specific expression in lymphoid tissues where immunoglobulin and T cell receptor gene rearrangements occur (Jeggo et al. in Bioessays (1995) 17:949-957), and in reproductive tissues where meiotic recombination take place. Consistent with such a role, is the increased gene expression observed in the G2-phase of the cell cycle, where mitotic recombination events during replication-coupled-repair occur (Hendrickson Am. J. Hum. Genet. (1997) 61:795-800). *Involvement in DNA Replication*

The role of NuSAP in DNA replication is supported by its localization adjacent to sites of BrdU incorporation. Replication factors that have been shown to localize to these sites include proliferating cell nuclear antigen (PCNA) (Bravo et al. in (J. Cell Biol. (1987)105:1549-54) and DNA ligase I (Montecucco et al. in EMBO J. (1995) 14:5379-5386). Interestingly, the cell cycle regulators cyclin A and Cdk 2 have also been shown to localize to these sites and they provide a direct link between cell cycle regulation and DNA replication (Cardoso et al. in Cell. (1993) 74:979-992). Localization of NuSAP adjacent to sites of BrdU incorporation is however partial: NuSAP foci are more abundant than replication foci, which indicates that not all NuSAP is participating in the replication process. NuSAPs involvement in DNA replication is also supported by the following observations. First, cell synchronization experiments reveal that *nusap* expression is upregulated during the replicative phase of the cell cycle, as determined by examining *histone H4* gene expression, an S-phase specific marker (Stein et al. in Curr. Opin. Cell Biol. (1992) 4:166-173). Second, *nusap* gene expression shows a strong correlation with the degree of cell proliferation (or DNA replication). In serum starvation experiments, *nusap* RNA levels declined when growth factors were withdrawn, and increased upon readdition. Similarly hydroxyurea treated cells showed a rapid decline in RNA levels of both *nusap* and *histone H4.* Third, the tissue-specific expression often shows a correlation with *histone H4* expression, a molecular marker whose expression is linked to DNA replication. In addition, the in situ data reveal that the cell-specific expression correlates with proliferative cells. In the thymus, expression is restricted to the immature T-stem cells which are actively dividing and in the embryonic small intestine, high NuSAP expression is restricted to the actively dividing cells in the crypts. Finally, sense and antisense studies in cultured cells show respectively an increase (COS-1) and decrease (NIH3T3 and COS-1) of [³H]-thymidine incorporation into cellular DNA, a reflection of DNA replication.

With respect to the exact function of NuSAP within the complex process of DNA replication, some cells that were in S-phase, as monitored by BrdU incorporation, showed that NuSAP was not localized to these intra-nuclear foci, indicating that this association is restricted to a certain period of the S-phase. In addition, participation in other fundamental cellular events like transcription and splicing is not excluded. These processes also occur within distinct domains in the nucleus (Berezney et al. in Int. Rev. Cytol. (1995) 162A:1-65) and the NuSAP foci that do not co-localize to sites of DNA replication may participate in these processes.

### Vitamin D Actions Linked to DNA Replication

The possible role of NuSAP in promoting DNA replication also provides a new mechanism underlying the anti-proliferative action of 1α,25(OH)₂D₃. To date these effects of 1α,25(OH)₂D₃ have been assigned to its induction of several cyclin-dependant kinase inhibitors, including p21 and p27 (Liu et al. in Genes Dev. (1996) 10:142-153.; Segaert et al. in J. Invest. Dermatol. (1997) 109:46-54), either directly or indirectly, and to its alteration of cyclin levels (Wang et al. in Cancer Res. (1996) 56:264-247; Verlinden Mol. Cell. Endocrinol. (1998) 142:57-65), ultimately resulting in an arrest of the cell cycle in the G1 phase. The present data show that 1α,25(OH)₂D₃ significantly down-regulates *nusap* expression 24 hours after treatment when DNA replication declines. *Possible Function in DNA Recombination*

The data obtained, however also show that NuSAP has additional roles besides DNA replication. From the tissue-specific expression pattern of *nusap* it is evident that its expression not always correlates with *histone H4* expression. The central nervous system and the kidney show higher RNA levels for *histone H4* than for NuSAP, while in the testis the opposite is observed. In addition, the cell-specific expression pattern in the testis and ovary further points to a role for NuSAP in the meiotic process. In the adult testis, high expression is confined to the primary spermatocytes in the late pachytene stage of meiosis I. During pachytene, the homologous chromosomes which are in synapsis remain closely associated until recombination is completed (Moens Bioessays. (1994) 16:101-106). The examples show that NuSAP is involved in the recombination event. Further support for this role is the high expression of NuSAP in the thymus, spleen and lymph node, where the gene product is considered to be involved in lymphoid cell-specific reactions such as recombination during immunoglobulin and T cell receptor gene rearrangements (Jeggo Bioessays. (1995) 17:949-957) and/ or immunoglobulin class switching (Harriman et al. in Annu. Rev. Immunol. (1993) 11:361-384). Furthermore, the high expression of *nusap* observed during the G2-phase of the cell cycle implies a role in DNA repair coupled to DNA replication: during the late S- and the G2-phase, the ploidy of the cell increases to 4n, allowing homologous recombination to proceed of an undamaged template (Hendrickson Am. J. Hum. Genet. (1997) 61:795-800). Interestingly, some nucleolar proteins have been linked to cellular recombination events: B23 (nucleophosmin) and nucleolin (C23) as part of the SWAP complex in B-cell switch recombination (Blose et al. in J. Cell Biol. (1984) 98:847-858), SC65 in meiotic recombination (Ochs et al. in Mol. Biol. Cell. (1996) 7:1015-1024), and Sir2 and Pch2 in yeast rDNA and meiotic recombination (San-Segundo et al. in Cell. (1999) 97:313-324). Recently, proteins with functions in both replication and recombination have been identified (Beernink et al. in Trends Biochem. Sci. (1999) 24:385-389; Kodadek, Trends Biochem. Sci. (1998) 23:79-83), although studies have generally been focused on prokaryotes and lower eukaryotes. A plausible working hypothesis is that these proteins bind to single stranded DNA (ssDNA) facilitating hereby the assembly of enzyme complexes (for DNA replication and recombination) to the ssDNA (Beernink et al., cited *supra*).

### Recruitment to the Nucleolus

During the interphase of proliferating cells, NuSAP localizes predominantly to the nucleolus. This was confirmed through colocalization studies with nucleolin, a major nucleolar protein. The nucleolus has long been believed to be a site solely dedicated to rDNA transcription and ribosome biogenesis (reviewed in Shaw et al. (1995) Annu. Rev. Cell Dev. Biol. 11:93-121). Recently however, additional nucleolar functions have been recognized (Pederson (1998) J. Cell Biol. 143:279-281) especially in yeast where mitotic and meiotic roles for the nucleolus in cell cycle control and transcriptional regulation have been defined (reviewed in Garcia et al. (1999) Cell. 97:825-828). The emerging view of these studies is that the nucleolus serves as a privileged site for both the recruitment and exclusion of regulatory complexes. NuSAP can in a similar way be recruited to the nucleolus and released when required in processes like DNA replication. NuSAP's interaction with nucleolin has been demonstrated. Its close association with the nucleolar remnant within the nuclear matrix, and its lack of association with extractable DNA and RNA within the nucleolus, further supports this. Such a recruitment will make a rapid response possible, independent of the transcriptional and translational regulatory circuits. The multiple consensus phosphorylation sites within NuSAP indicates that such a response can be triggered by phosphorylations. Interestingly, a role in the initiation of DNA replication has been assigned to nucleolin, based on its ability to form a complex with Replication Protein A after cell stress (Daniely et al. (2000) J. Cell Biol. 149, 799-809).

### Association wit the nuclear matrix

The protein was named NuSAP based on the initial observation of being a nuclear spindle-associated protein. The strong conservation of protein sequence in vertebrates indicates an essential function in higher eukaryotes. A structural role within the nucleus is proposed, based on the localization of the mouse protein to an unique intranuclear filamentous network. Its organization within the nucleus is reminiscent of the structural framework observed in nuclear matrix preparations (He et al. (1990) J. Cell Biol. 110:569-580; Nickerson et al. (1995) Int. Rev. Cytol. 162A:67-123; Wan et al. (1999) Proc. Natl. Acad. Sci. USA. 96: 933-938). The nuclear matrix is the non-chromatin nuclear structure that likely serves as a scaffold, determining the general organization of the nucleus. By positioning and transporting nuclear factors to appropriate spatial domains within the nucleus, the nuclear matrix is likely to contribute to the efficient assembly of functional complexes. The molecular composition of the structural components of the nuclear matrix has not yet been clearly determined, but candidates do exist. The filament-forming Tpr protein is one such candidate. It is a nuclear pore complex associated protein that is organized into filaments extending 100-350 nm into the nucleus (Cordes et al. (1993) J. Cell Biol. 123:1333-1344; Zimowska et al. (1997) J. Cell Sci. 110, 927-944; Strambio-de-Castillia et al. (1999) J. Cell Biol. 144: 839-855). It has also been shown to have a function in nuclear trafficking of macromolecules (Strambio-de-Castillia et al. , cited supra; Bangs et al. (1998) J. Cell Biol. 143: 1801-1812). A second group of candidates composing the nuclear matrix are the nuclear lamins. They not only form a filamentous structure under the nuclear envelope, but also exist as foci within the nuclear interior. However, the presence of an extensive internal lamin network has yet to be demonstrated. Our observations of the distribution pattern of NuSAP indicate that it is clearly distinct from the filament-forming proteins mentioned above. As expected, the filaments also proved to be distinct to actin filaments and microtubules. At the core of the nucleus NuSAP's localization to filaments is less evident, but some filaments remain discernible. Electron microscopic analysis will bring clarification to this issue. Previous studies have suggested that the nuclear matrix helps coordinate the spatial organization of DNA replication (Berezney et al. (1981) Exp. Cell Res. 132:1-13; Vaughn et al. (1990) Nucleic Acids Res. 18:1965-1969). NuSAP's localization to multiple small foci embedded within the network, which are seen adjacent to sites of newly replicated DNA, suggest a role for the protein in the replication process

### Association with the Mitotic Spindle and Chromosomes

With respect to the functional significance of NuSAPs association with different cellular structures (condensing chromosomes during prophase, mitotic spindle during metaphase and anaphase, and the chromosomes and midbody during telophase) during mitosis the multiple associations form a pathway through which daughter cells are ensured equal amounts of NuSAP. Alternatively, association to the respective cellular structures is of functional importance and would imply a critical role for NuSAP in determining progression of the cell cycle. At the start of mitosis, nucleolar NuSAP translocates to the condensing chromosomes. It has been shown that histone H1, with a possible chromosome condensing activity (Bradbury et al. (1974) Nature. 247:257-261), becomes phosphorylated by Cdc 2 kinase as cells enter mitosis (Langan et al. (1989) Mol. Cell. Biol. 9:3860-3868). NuSAP contains three consensus Cdc2 phosphorylation sites that were characterized by Peter et al. (1990) Cell. 60:791-801 based on Cdc2 phosphorylation of nucleolin during mitosis. The existence of consensus Cdc 2 phosphorylation sites in NuSAP may be linked to its association with condensing chromosomes during mitosis. During metaphase and anaphase, the association of NuSAP with the mitotic spindle and its poles is particularly evident. This association with microtubules is sensitive to treatment with nocodazole, a microtubule depolymerizer (Jordan et al. (1992) J. Cell Sci. 102:401-416) and is induced by treatment of mitotic cells with taxol, a microtubule stabilizer capable of inducing microtubule asters (De Brabander et al. in Proc. Natl. Acad. Sci. USA. 78:5608-5612).

Taken together, we show that NuSAP is a novel nuclear protein that has a role in DNA replication and most likely in other cellular processes including recombination. Based on the highly basic nature of the protein, we assign to NuSAP a structural role in these processes. The multiple consensus phosphorylation sites make its regulation during these processes possible.

## Claims

1. An isolated and purified eukaryotic polynucleotide encoding a Nuclear Spindle-Associating Protein, the said polynucleotide containing the nucleic acid sequence shown in SEQ ID NO:1 or the nucleic acid sequence shown in SEQ ID NO:3, or an allelic variant thereof.

2. A Nuclear Spindle-Associating Protein having the amino acid sequence SEQ ID NO:2 or the amino acid sequence SEQ ID NO:4, or a protein having at least 90 % amino acid sequence identity to said protein having the amino acid sequence of SEQ ID NO:2 or SEQ ID NO 4 or a protein encoded by an allelic variant of a polynucleotide containing the nucleic acid sequence shown in SEQ ID NO:1 or the nucleic acid sequence shown in SEQ ID NO:3.

3. A eukaryotic polynucleotide according to claim 1, further comprising a polynucleotide which codes for at least a portion of a protein or a marker, the combination of both polynucleotides being arranged in such a way that a fusion protein results after expression.

4. A vector comprising at least a eukaryotic polynucleotide according to claim 1 or 3.

5. A fusion protein encoded by the polynucleotide according to claim 3.

6. Use of a eukaryotic polynucleotide according to claim 1, or of a probe capable of specifically hybridizing to the polynucleotide of claim 1 or specifically amplifying a sequence within the polynucleotide of claim 1, or of a protein according to claim 2 or a portion thereof, or of a fusion protein according to claim 5 for the manufacture of a diagnostic tool for the detection of cancer of a tissue selected from the group consisting of kidney, stomach, small intestine, lung, ovary, cervix, breast and uterus.

7. Use of a polynucleotide according to claim 1 or a protein according to claim 2 or a fusion protein according to claim 5 for the manufacture of a diagnostic tool.

8. A pharmaceutical composition comprising a eukaryotic polynucleotide according to claim 1, and a pharmaceutically acceptable carrier.

9. A process for the production of a protein according to claim 2, comprising culturing a host cell containing an expression vector comprising a eukaryotic polynucleotide according to claim 1, and obtaining said protein from the cell or the culture medium.

10. A host cell, other than a human germ line cell, being transformed with a eukaryotic polynucleotide according to claim 1 or 3.

11. A transgenic rodent whose genome is heterozygous for an engineered disruption in a rodent NuSAP gene coding for SEQ ID NO:1 or its rodent homologue, wherein said engineered disruption in a heterozygous state inhibits overall or tissue specific production of a NuSAP protein having the amino acid sequence SEQ ID NO:2 or the rodent homologue thereof, thereby resulting in a transgenic rodent which has increased tendency, with respect to a wild-type rodent, to disorders associated with DNA replication, cell proliferation and meiosis.

12. A transgenic rodent according to claim 11, further comprising a promoter in front of the gene for modulating the expression of the protein in comparison with the wild-type.

13. A ribozyme capable of cleaving the mRNA of a protein according to claim 2, the said ribozyme including sequences complementary to portions of mRNA obtained from a eukaryotic polynucleotide according to claim 1.

14. An antisense oligonucleotide capable of blocking expression of a eukaryotic polynucleotide according to claim 1.

15. A vector which produces in a cell, other than a human germ line cell, the ribozyme of claim 13 or the antisense oligonucleotide of claim 19.

16. A monoclonal antibody being able to discriminate between a Nuclear Spindle-Associating Protein according to claim 2 with and without a post-translationally modified amino-acid.

17. A eukaryotic polynucleotide according to claim 1 for use as a medicament.

18. Use of a eukaryotic polynucleotide according to claim 1 for the manufacture of a medicament for the prevention or treatment of a disorder associated with DNA replication, cell proliferation or meiosis.

19. The use according to claim 18, wherein said disorder is a disorder associated with cell hyperproliferation.

20. The use according to claim 18 or 19, wherein said disorder is cancer.

21. The use according to claim 20, wherein said disorder is a cancer of the tissue selected from the group consisting of kidney, stomach, small intestine, lung, ovary, cervix, breast and uterus.

22. The use according to claim 18, wherein said disorder is a disorder of reproduction in eukaryotic cells.

23. The use to claim 18, wherein the said disorder is a disorder of the immune system.

24. The use according to claim 18, wherein the said disorder is a disorder related to bones of mammals.

25. The use according to claim 18, wherein the said disorder is a disorder caused by mutations in nuclear structural proteins, such as Emery-Dreifuss muscular distrophy, dilated cardiomyopathy or lipodistrophy.

## Patentansprüche

1. Isoliertes und gereinigtes eukaryontisches Polynukleotid, welches für ein Kernspindel-assoziiertes Protein kodiert, wobei das Polynukleotid die Nukleinsäuresequenz von SEQ ID NO: 1 oder die Nukleinsäuresequenz von SEQ ID NO:3 oder eine Allelvariante davon enthält.

2. Kernspindel-assoziiertes Protein mit der Aminosäuresequenz SEQ ID NO:2 oder der Aminosäuresequenz SEQ ID NO:4 oder ein Protein mit zumindest 90% Aminosäuresequenzidentität zum Protein mit der Aminosäuresequenz SEQ ID NO:2 oder SEQ ID NO:4 oder ein Protein, das durch eine Allelvariante eines Polynukleotids kodiert wird, enthaltend die Nukleinsäuresequenz von SEQ ID NO:1 oder die Nukleinsäuresequenz von SEQ ID NO:3.

3. Eukaryontisches Polynukleotid nach Anspruch 1, weiterhin umfassend ein Polynukleotid, welches für zumindest einen Teil eines Proteins oder eines Markers kodiert, wobei die Kombination beider Polynukleotide derart angeordnet ist, dass nach Expression ein Fusionsprotein resultiert.

4. Vektor, umfassend zumindest ein eukaryontisches Polynukleotid nach Anspruch 1 oder 3.

5. Fusionsprotein, kodiert durch das Polynukleotid nach Anspruch 3.

6. Verwendung eines eukaryontischen Polynukleotids nach Anspruch 1, oder einer Sonde, die zur spezifischen Hybridisierung an das Polynukleotid von Anspruch 1 oder zur spezifischen Amplifikation einer Sequenz mit dem Polynukleotid nach Anspruch 1 befähigt ist, oder eines Proteins nach Anspruch 2 oder eines Teils hiervon, oder eines Fusionsproteins nach Anspruch 5 zur Herstellung eines diagnostischen Werkzeugs zum Nachweis von Krebs bei einem Gewebe, ausgewählt aus der Gruppe, bestehend aus Niere, Magen, Dünndarm, Lunge, Eierstock, Cervix, Brust und Uterus.

7. Verwendung eines Polynukleotids nach Anspruch 1 oder eines Proteins nach Anspruch 2 oder eines Fusionsproteins nach Anspruch 5 zur Herstellung eines diagnostischen Werkzeugs.

8. Pharmazeutische Zusammensetzung, umfassend ein eukaryontisches Polynukleotid nach Anspruch 1 und einen pharmazeutisch verträglichen Träger.

9. Verfahren zur Herstellung eines Proteins nach Anspruch 2, umfassend das Kultivieren einer Wirtszelle, die einen Expressionsvektor mit einem eukaryontischen Polynukleotid nach Anspruch 1 enthält, und Gewinnen des Proteins aus der Zelle oder dem Kulturmedium.

10. Wirtszelle, die keine Human-Keimbahnzelle ist, transformiert mit einem eukaryontischen Polynukleotid nach Anspruch 1 oder 3.

11. Transgener Nager, dessen Genom heterozygot in Bezug auf eine eingeführte Disruption in einem NuSAP-Nagergen, welches für SEQ ID NO:1 kodiert, ist oder sein Nager-Homolog, wobei die eingeführte Disruption in einem heterozygoten Zustand insgesamt oder gewebespezifisch die Produktion eines NuSAP-Proteins mit der Aminosäuresequenz SEQ ID NO:2 oder des Nager-Homologs hiervon hemmt, wodurch ein transgener Nager erhalten wird, der, bezogen auf einen Wildtyp-Nager, eine erhöhte Neigung zu Funktionsstörungen der DNA-Replikation, der Zellproliferation und der Meiose besitzt.

12. Transgener Nager nach Anspruch 11, weiterhin umfassend einen Promotor vor dem Gen zur Modulation der Expression des Proteins im Vergleich zum Wildtyp.

13. Ribozym, das zum Schneiden der mRNA eines Proteins nach Anspruch 2 befähigt ist, wobei das Ribozym Sequenzen enthält, die zu Anteilen der mRNA komplementär sind, die aus einem eukaryontischen Polynukleotid nach Anspruch 1 erhalten wurden.

14. Antisense-Oligonukleotid, das zum Blockieren der Expression eines eukaryontischen Polynukleotids nach Anspruch 1 befähigt ist.

15. Vektor, der in einer Zelle, die nicht eine Human-Keimbahnzelle ist, das Ribozym nach Anspruch 13 oder das Anitsense-Oligonukleotid nach Anspruch 19 produziert.

16. Monoklonaler Antikörper, der zur Unterscheidung zwischen einem Kernspindelassoziertem Protein nach Anspruch 2 mit und ohne einer posttranslational modifizierten Aminosäure befähigt ist.

17. Eukaryontisches Polynukleotid nach Anspruch 1 zur Verwendung als Medikament.

18. Verwendung eines eukaryontischen Polynukleotids nach Anspruch 1 zur Herstellung eines Medikaments zur Prävention oder Behandlung einer Erkrankung, die mit der DNA-Replikation, Zellproliferation oder Meiose assoziiert ist.

19. Verwendung nach Anspruch 18, wobei die Erkrankung eine mit einer Zellhyperproliferation assoziierte Erkrankung ist.

20. Verwendung nach Anspruch 18 oder 19, wobei die Erkrankung Krebs ist.

21. Verwendung nach Anspruch 20, wobei die Erkrankung ein Krebs des Gewebes ist, ausgewählt aus der Gruppe, bestehend aus Niere, Magen, Dünndarm, Lunge, Eierstock, Cervix, Brust und Uterus.

22. Verwendung nach Anspruch 18, wobei die Erkrankung eine Erkrankung der Reproduktion in eukaryontischen Zellen ist.

23. Verwendung nach Anspruch 18, wobei die Erkrankung eine Erkrankung des Immunsystems ist.

24. Verwendung nach Anspruch 18, wobei die Erkrankung eine Erkrankung ist, die in Verbindung mit Knochen von Säugern steht.

25. Verwendung nach Anspruch 18, wobei die Erkrankung eine Erkrankung ist, die durch Mutationen in Kernstrukturproteinen verursacht wird, beispielsweise die Emery-Dreifuss-Muskeldystrophie, Cardiomyopathie-Dilatation oder Lipodystrophie.

## Revendications

1. Polynucléotide eucaryote isolé et purifié codant pour une protéine nucléaire s'associant au fuseau, ledit polynucléotide contenant la séquence d'acide nucléique représentée dans SEQ ID NO: 1 ou la séquence d'acide nucléique représentée dans SEQ ID NO: 3, ou un variant allélique de celles-ci.

2. Protéine nucléaire s'associant au fuseau possédant la séquence d'acides aminés SEQ ID NO: 2 ou la séquence d'acides aminés SEQ ID NO: 4, ou protéine ayant au moins 90 % d'identité de séquence d'acides aminés avec ladite protéine possédant la séquence d'acides aminés de SEQ ID NO: 2 ou de SEQ ID NO: 4 ou avec une protéine codée par un variant allélique d'un polynucléotide contenant la séquence d'acide nucléique représentée dans SEQ ID NO: 1 ou la séquence d'acide nucléique représentée dans SEQ ID NO: 3.

3. Polynucléotide eucaryote selon la revendication 1, comprenant en outre un polynucléotide qui code pour au moins une partie d'une protéine ou d'un marqueur, la combinaison des deux polynucléotides étant organisée d'une manière telle qu'il en résulte une protéine de fusion après l'expression.

4. Vecteur comprenant au moins un polynucléotide eucaryote selon la revendication 1 ou 3.

5. Protéine de fusion codée par le polynucléotide selon la revendication 3.

6. Utilisation d'un polynucléotide eucaryote selon la revendication 1, ou d'une sonde capable de s'hybrider spécifiquement au polynucléotide de la revendication 1 ou d'amplifier spécifiquement une séquence du polynucléotide de la revendication 1, ou d'une protéine selon la revendication 2 ou d'une partie de celle-ci, ou d'une protéine de fusion selon la revendication 5 pour la fabrication d'un outil de diagnostic destiné à la détection du cancer d'un tissu choisi dans le groupe constitué par le rein, l'estomac, l'intestin grêle, le poumon, l'ovaire, le col, le sein et l'utérus.

7. Utilisation d'un polynucléotide selon la revendication 1 ou d'une protéine selon la revendication 2 ou bien d'une protéine de fusion selon la revendication 5 pour la fabrication d'un outil de diagnostic.

8. Composition pharmaceutique comprenant un polynucléotide eucaryote selon la revendication 1, et un support pharmaceutiquement acceptable.

9. Procédé pour la production d'une protéine selon la revendication 2, comprenant la culture d'une cellule hôte contenant un vecteur d'expression comprenant un polynucléotide eucaryote selon la revendication 1, et obtention de ladite protéine à partir de la cellule ou du milieu de culture.

10. Cellule hôte, autre qu'une cellule de la lignée germinale humaine, qui est transformée avec un polynucléotide eucaryote selon la revendication 1 ou 3.

11. Rongeur transgénique dont le génome est hétérozygote pour une interruption obtenue par manipulation dans un gène de NuSAP de rongeur codant pour SEQ ID NO: 1 ou son homologue pour les rongeurs, chez lequel ladite interruption obtenue par manipulation dans un état hétérozygote inhibe l'ensemble de la production ou la production spécifique d'un tissu d'une protéine NuSAP possédant la séquence d'acides aminés SEQ ID NO: 2 ou l'homologue de celle-ci pour les rongeurs, en ayant ainsi pour résultat un rongeur transgénique qui a une tendance accrue, par rapport à un rongeur de type sauvage, à des troubles associés à la réplication de l'ADN, à la prolifération cellulaire et à la méiose.

12. Rongeur transgénique selon la revendication 11, comprenant en outre un promoteur devant le gène permettant de moduler l'expression de la protéine par comparaison avec le type sauvage.

13. Ribozyme capable de cliver l'ARNm d'une protéine selon la revendication 2, ledit ribozyme comprenant des séquences complémentaires de parties d'ARNm obtenues à partir d'un polynucléotide eucaryote selon la revendication 1.

14. Oligonucléotide antisens capable de bloquer l'expression d'un polynucléotide eucaryote selon la revendication 1.

15. Vecteur qui produit dans une cellule, autre qu'une cellule de la lignée germinale humaine, le ribozyme de la revendication 13 ou l'oligonucléotide antisens de la revendication 14.

16. Anticorps monoclonal qui est capable de faire la distinction entre une protéine nucléaire s'associant au fuseau selon la revendication 2 qui possède un acide aminé modifié après la traduction et une protéine qui en est dépourvue.

17. Polynucléotide eucaryote selon la revendication 1 destiné à être utilisé en tant que médicament.

18. Utilisation d'un polynucléotide eucaryote selon la revendication 1 pour la fabrication d'un médicament destiné à la prévention ou au traitement d'un trouble associé à la réplication de l'ADN, à la prolifération cellulaire ou à la méiose.

19. Utilisation selon la revendication 18, dans laquelle ledit trouble est un trouble associé à une hyperprolifération cellulaire.

20. Utilisation selon la revendication 18 ou 19, dans laquelle ledit trouble est un cancer.

21. Utilisation selon la revendication 20, dans laquelle ledit trouble est un cancer du tissu choisi dans le groupe constitué par le rein, l'estomac, l'intestin grêle, le poumon, l'ovaire, le col, le sein et l'utérus.

22. Utilisation selon la revendication 18, dans laquelle ledit trouble est un trouble de la reproduction dans des cellules eucaryotes.

23. Utilisation selon la revendication 18, dans laquelle ledit trouble est un trouble du système immunitaire.

24. Utilisation selon la revendication 18, dans laquelle ledit trouble est un trouble lié aux os des mammifères.

25. Utilisation selon la revendication 18, dans laquelle ledit trouble est un trouble dû à des mutations dans des protéines nucléaires de structure, tel que la dystrophie musculaire d'Emery-Dreifuss, une cardiomyopathie dilatée ou une lipodystrophie.


### SEQUENCE LISTING

<110> K U Leuven Research and Development
   Bouillon, Roger
   Carmeliet, Geert
   Raemaekers, Tim
<120> nusap, a novel gene encoding a tissue-specific nuclear protein, useful as a diagnositc tool and therapeutic agent
<130> L-1764-pct
<140>
   <141>
<150> US 60/189,743
   <151> 2000-03-16
<160> 4
<170> PatentIn Ver. 2.1
<210> 1
   <211> 2136
   <212> DNA
   <213> Mus musculus
<220>
   <221> CDS
   <222> (71)..(1354)
   <223> putative SAP domain AA 9-43 , putative NLS : AA 203-220
<220>
   <221> polyA_signal
   <222> (1885)..(1890)
<220>
   <221> polyA_signal
   <222> (2123)..(2127)
<400> 1
   ccagttttga gccgctctgt tttgagttgt gtggctgttt ttctgggaat caccgagatt 60
   gcagaacgcg atg acc gtc ccc tct gca gag gag ctg gac tcc ttt aag    109
              Met Thr Val Pro Ser Ala Glu Glu Leu Asp Ser Phe Lys
                1               5                  10
   tac agc gac ctg caa aat tta gcc aaa agg ctg ggc ctc cgg gct aac   157
   Tyr Ser Asp Leu Gln Asn Leu Ala Lys Arg Leu Gly Leu Arg Ala Asn
        15                  20                  25
   atg aag gca gac aag ttg tta aaa gcc ttg aaa gca cac ctg aat cca   205
   Met Lys Ala Asp Lys Leu Leu Lys Ala Leu Lys Ala His Leu Asn Pro
    30                  35                  40                  45
   gaa aca agg aaa gaa aat aaa aat cag gat gaa aat caa ttc tcc act   253
   Glu Thr Arg Lys Glu Asn Lys Asn Gln Asp Glu Asn Gln Phe Ser Thr
                    50                  55                  60
   gat gaa act gag ata cac gtt agc agt gag gag caa gct gag acg gaa   301
   Asp Glu Thr Glu Ile His Val Ser Ser Glu Glu Gln Ala Glu Thr Glu
                65                  70                  75
   tca ggt ggt cac gtc acc aaa acg agg agg agg agg agg aag aag cac   349
   Ser Gly Gly His Val Thr Lys Thr Arg Arg Arg Arg Arg Lys Lys His
            80                  85                  90
   aag acc atc cat gga att cct acc tcc cag act ttg ttg cag gat cat   397
   Lys Thr Ile His Gly Ile Pro Thr Ser Gln Thr Leu Leu Gln Asp His
        95                 100                 105
   ttg gag atg aaa gga act gat agt agt aac ttc caa aat caa gaa aat   445
   Leu Glu Met Lys Gly Thr Asp Ser Ser Asn Phe Gln Asn Gln Glu Asn
   110                 115                 120                 125
   cag gaa aat caa gac ccc agg gat aca gca gaa gtt cct tct ctg ccg   493
   Gln Glu Asn Gln Asp Pro Arg Asp Thr Ala Glu Val Pro Ser Leu Pro
                   130                 135                 140
   gag cag agg cca gag gac ggc aat gcg gct tct tca gga gaa gga gaa   541
   Glu Gln Arg Pro Glu Asp Gly Asn Ala Ala Ser Ser Gly Glu Gly Glu
               145                 150                 155
   gta aat gac att aaa gat tca aag aag cct tta gaa aaa aga tct cta   589
   Val Asn Asp Ile Lys Asp Ser Lys Lys Pro Leu Glu Lys Arg Ser Leu
           160                 165                 170
   tgc acg gat gag ttt tct aaa ctt ggg aac aat aaa agg act tca gcc   637
   Cys Thr Asp Glu Phe Ser Lys Leu Gly Asn Asn Lys Arg Thr Ser Ala
       175                 180                 185
   aca aca cca aac ttt aag aag ctt cat gag gct cgt ttt aag aaa atg   685
   Thr Thr Pro Asn Phe Lys Lys Leu His Glu Ala Arg Phe Lys Lys Met
   190                 195                 200                 205
   gaa tcc att gat gaa tat att atg agg aaa aag aaa cac ctt aaa gaa    733
   Glu Ser Ile Asp Glu Tyr Ile Met Arg Lys Lys Lys His Leu Lys Glu
                   210                 215                 220
   cac agt tca ctt aat gaa cta aag ctt gac aaa aaa ggg ata gtg acc    781
   His Ser Ser Leu Asn Glu Leu Lys Leu Asp Lys Lys Gly Ile Val Thr
               225                 230                 235
   cca gtt cct cca aga gga agg ctc tct gta ccc tgt act cct gcc agg    829
   Pro Val Pro Pro Arg Gly Arg Leu Ser Val Pro Cys Thr Pro Ala Arg
           240                 245                 250
   cag cag tgc cca caa ggc cac tca gca act aaa atg aat gtc agg ttt    877
   Gln Gln Cys Pro Gln Gly His Ser Ala Thr Lys Met Asn Val Arg Phe
       255                 260                 265
   tca gct gct act aaa gac aat gaa cat aag tgc tca ctg acc aag aca    925
   Ser Ala Ala Thr Lys Asp Asn Glu His Lys Cys Ser Leu Thr Lys Thr
   270                 275                 280                 285
   cca gcc aga aag tct cca cac gtg act gca cct ggg agt gct tca aaa    973
   Pro Ala Arg Lys Ser Pro His Val Thr Ala Pro Gly Ser Ala Ser Lys
                   290                 295                 300
   ggc cag gct gtg ttc agg aca ccc aag tca aag gcc act gaa agg act    1021
   Gly Gln Ala Val Phe Arg Thr Pro Lys Ser Lys Ala Thr Glu Arg Thr
               305                 310                 315
   tct att gca gtt att acc cct ttc aag ttg atg act gaa gca aca cag    1069
   Ser Ile Ala Val Ile Thr Pro Phe Lys Leu Met Thr Glu Ala Thr Gln
           320                 325                 330
   act cca agt tct agt aag aag cca gta ttt gat ctc aaa gca agc ttg    1117
   Thr Pro Ser Ser Ser Lys Lys Pro Val Phe Asp Leu Lys Ala Ser Leu
       335                 340                 345
   tct cgt ccc ctc aac tac aag cca cac aaa gga gag ctg aaa cct tgg    1165
   Ser Arg Pro Leu Asn Tyr Lys Pro His Lys Gly Glu Leu Lys Pro Trp
   350                 355                 360                 365
   gga caa gct aaa gag aac aat tct ctg aac gaa cgt gta agc aga gtt    1213
   Gly Gln Ala Lys Glu Asn Asn Ser Leu Asn Glu Arg Val Ser Arg Val
                   370                 375                 380
   acc ttc cac agg aaa act tac aaa caa cct cat ctc caa acc agg gaa    1261
   Thr Phe His Arg Lys Thr Tyr Lys Gln Pro His Leu Gln Thr Arg Glu
               385                 390                 395
   gaa cga tgg aag aga caa gag caa gaa cga aag gag aag aaa gaa aag     1309
   Glu Arg Trp Lys Arg Gln Glu Gln Glu Arg Lys Glu Lys Lys Glu Lys
            400                 405                 410
   ctt ttg gaa gct cga aga aac ctg ggt gtg act aaa gcc cag tga         1354
   Leu Leu Glu Ala Arg Arg Asn Leu Gly Val Thr Lys Ala Gln
        415                 420                 425
   ccctgtctgt tcctttactc taacttgttt tccttttgta tgttttttac tctttctcta 1414
   cttcagtcaa aagctctttt ctatcataac ttttggtcat aatttgtgta gtgtctcttc 1474
   tgtgctatat ctgagaatat attatcacct taaagttcat aactaaagta gttttcatct 1534
   aatgccctat acatctccaa tttttaaaag acttgtcctc atgattctta acagaggttt 1594
   ttcatgtcaa ggtcctgata gttccgaggg agatgacctg cacccttttc tgcacaagct 1654
   gtagcagaat cttagcatga gaaaataaga tatcgctcat aggaaaaggg aggctggaag 1714
   accacatttt tgttcagtag cctggaaaat ctattagtct tattgaaatc ttattttatc 1774
   aaggtaaaaa gttttagctt ataagagcct tgttctgact tttcatgtat ttgccatctg 1834
   tcattcatta taatcctaaa cgagaaaacc tctactctct ctacttgtta aataaaagcc 1894
   acagggcaaa gggtgtgctt tagttgtaca acacttgtct atcacactcc tgtccctgaa 1954
   ctgtaccacc aaaaccaaag ctgagaattg ctgctgtaag aattactgtc attggcagac 2014
   tttttcttta caagtagtaa agagaggaaa gctgcaaggg gtgaccttct gatctttgct 2074
   ctgccttaca gagattctga gatgtgtgta atgcttacaa tgttcataaa taaaaatttt 2134
   ca                                                                2136
<210> 2
   <211> 427
   <212> PRT
   <213> Mus musculus
<400> 2
   Met Thr Val Pro Ser Ala Glu Glu Leu Asp Ser Phe Lys Tyr Ser Asp
   1 5 10 15
   Leu Gln Asn Leu Ala Lys Arg Leu Gly Leu Arg Ala Asn Met Lys Ala
   20 25 30
   Asp Lys Leu Leu Lys Ala Leu Lys Ala His Leu Asn Pro Glu Thr Arg
   35 40 45
   Lys Glu Asn Lys Asn Gln Asp Glu Asn Gln Phe Ser Thr Asp Glu Thr
   50 55 60
   Glu Ile His Val Ser Ser Glu Glu Gln Ala Glu Thr Glu Ser Gly Gly
   65 70 75 80
   His Val Thr Lys Thr Arg Arg Arg Arg Arg Lys Lys His Lys Thr Ile
   85 90 95
   His Gly Ile Pro Thr Ser Gln Thr Leu Leu Gln Asp His Leu Glu Met
   100 105 110
   Lys Gly Thr Asp Ser Ser Asn Phe Gln Asn Gln Glu Asn Gln Glu Asn
   115 120 125
   Gln Asp Pro Arg Asp Thr Ala Glu Val Pro Ser Leu Pro Glu Gln Arg
   130 135 140
   Pro Glu Asp Gly Asn Ala Ala Ser Ser Gly Glu Gly Glu Val Asn Asp
   145 150 155 160
   Ile Lys Asp Ser Lys Lys Pro Leu Glu Lys Arg Ser Leu Cys Thr Asp
   165 170 175
   Glu Phe Ser Lys Leu Gly Asn Asn Lys Arg Thr Ser Ala Thr Thr Pro
   180 185 190
   Asn Phe Lys Lys Leu His Glu Ala Arg Phe Lys Lys Met Glu Ser Ile
   195 200 205
   Asp Glu Tyr Ile Met Arg Lys Lys Lys His Leu Lys Glu His Ser Ser
   210 215 220
   Leu Asn Glu Leu Lys Leu Asp Lys Lys Gly Ile Val Thr Pro Val Pro
   225 230 235 240
   Pro Arg Gly Arg Leu Ser Val Pro Cys Thr Pro Ala Arg Gln Gln Cys
   245 250 255
   Pro Gln Gly His Ser Ala Thr Lys Met Asn Val Arg Phe Ser Ala Ala
   260 265 270
   Thr Lys Asp Asn Glu His Lys Cys Ser Leu Thr Lys Thr Pro Ala Arg
   275 280 285
   Lys Ser Pro His Val Thr Ala Pro Gly Ser Ala Ser Lys Gly Gln Ala
   290 295 300
   Val Phe Arg Thr Pro Lys Ser Lys Ala Thr Glu Arg Thr Ser Ile Ala
   305 310 315 320
   Val Ile Thr Pro Phe Lys Leu Met Thr Glu Ala Thr Gln Thr Pro Ser
   325 330 335
   Ser Ser Lys Lys Pro Val Phe Asp Leu Lys Ala Ser Leu Ser Arg Pro
   340 345 350
   Leu Asn Tyr Lys Pro His Lys Gly Glu Leu Lys Pro Trp Gly Gln Ala
   355 360 365
   Lys Glu Asn Asn Ser Leu Asn Glu Arg Val Ser Arg Val Thr Phe His
   370 375 380
   Arg Lys Thr Tyr Lys Gln Pro His Leu Gln Thr Arg Glu Glu Arg Trp
   385 390 395 400
   Lys Arg Gln Glu Gln Glu Arg Lys Glu Lys Lys Glu Lys Leu Leu Glu
   405 410 415
   Ala Arg Arg Asn Leu Gly Val Thr Lys Ala Gln
                   420                 425
<210> 3
   <211> 1551
   <212> DNA
   <213> Homo sapiens
<220>
   <221> CDS
   <222> (69)..(1391)
   <223> putative SAP domain: AA 9-43 ; putative NLS : 194-211
<400> 3
   gggatttgaa ccncgctgac gaagtttggt gatccatctt ccgagtatcg ccgggatttc 60
   gaatcgcg atg atc atc ccc tct cta gag gag ctg gac tcc ctc aag tac  110
            Met Ile Ile Pro Ser Leu Glu Glu Leu Asp Ser Leu Lys Tyr
             1               5                   10
   agt gac ctg cag aac tta gcc aag agt ctg ggt ctc cgg gcc aac ctg   158
   Ser Asp Leu Gln Asn Leu Ala Lys Ser Leu Gly Leu Arg Ala Asn Leu
    15                  20                  25                  30
   agg gca acc aag ttg tta aaa gcc ttg aaa ggc tac att aaa cat gag   206
   Arg Ala Thr Lys Leu Leu Lys Ala Leu Lys Gly Tyr Ile Lys His Glu
                    35                  40                  45
   gca aga aag gga aat gag aat cag gat gaa agt caa act tct gca tcc   254
   Ala Arg Lys Gly Asn Glu Asn Gln Asp Glu Ser Gln Thr Ser Ala Ser
                50                  55                  60
   tct tgt gat gag act gag ata cag atc agc aac cag gaa gaa gct gag   302
   Ser Cys Asp Glu Thr Glu Ile Gln Ile Ser Asn Gln Glu Glu Ala Glu
            65                  70                  75
   aga cag cca ctt ggc cat gtc acc aaa aca agg aga agg tgc aag act   350
   Arg Gln Pro Leu Gly His Val Thr Lys Thr Arg Arg Arg Cys Lys Thr
        80                  85                  90
   gtc cgt gtg gac cct gac tca cag cag aat cat tca gag ata aaa ata   398
   Val Arg Val Asp Pro Asp Ser Gln Gln Asn His Ser Glu Ile Lys Ile
    95                 100                 105                 110
   agt aat ccc act gaa ttc cag aat cat gaa aag cag gaa agc cag gat   446
   Ser Asn Pro Thr Glu Phe Gln Asn His Glu Lys Gln Glu Ser Gln Asp
                   115                 120                 125
   ctc aga gct act gca aaa gtt cct tct cca cca gac gag cac caa gaa      494
   Leu Arg Ala Thr Ala Lys Val Pro Ser Pro Pro Asp Glu His Gln Glu
               130                 135                 140
   gct gag aat gct gtt tcc tca ggt aac aga gat tca aag gta cct tca      542
   Ala Glu Asn Ala Val Ser Ser Gly Asn Arg Asp Ser Lys Val Pro Ser
           145                 150                 155
   gaa gga aag aaa tct ctc tac aca gat gag tca tcc aaa cct gga aaa      590
   Glu Gly Lys Lys Ser Leu Tyr Thr Asp Glu Ser Ser Lys Pro Gly Lys
       160                 165                 170
   aat aaa aga act gca atc act act cca aac ttt aag aag ctt cat gaa      638
   Asn Lys Arg Thr Ala Ile Thr Thr Pro Asn Phe Lys Lys Leu His Glu
   175                 180                 185                 190
   gct cat ttt aag gaa atg gag tcc att gat caa tat att gag aga aaa      686
   Ala His Phe Lys Glu Met Glu Ser Ile Asp Gln Tyr Ile Glu Arg Lys
                   195                 200                 205
   aag aaa cat ttt gaa gaa cac aat tcc atg aat gaa ctg aag cag ccc      734
   Lys Lys His Phe Glu Glu His Asn Ser Met Asn Glu Leu Lys Gln Pro
               210                 215                 220
   atc aat aag gga ggg gtc agg act cca gta cct cca aga gga aga ctc      782
   Ile Asn Lys Gly Gly Val Arg Thr Pro Val Pro Pro Arg Gly Arg Leu
           225                 230                 235
   tct gtg gct tct act ccc atc agc caa cga cgc tcg caa ggc cgg tct      830
   Ser Val Ala Ser Thr Pro Ile Ser Gln Arg Arg Ser Gln Gly Arg Ser
       240                 245                 250
   tgt ggc cct gca agt cag agt acc ttg ggt ctg aag ggg tca ctc aag      878
   Cys Gly Pro Ala Ser Gln Ser Thr Leu Gly Leu Lys Gly Ser Leu Lys
   255                 260                 265                 270
   ccg tct gct atc tct gca gct aaa acg ggt gtc agg ttt tca gct gct      926
   Pro Ser Ala Ile Ser Ala Ala Lys Thr Gly Val Arg Phe Ser Ala Ala
                   275                 280                 285
   act aaa gat aat gag cat aag cgt tca ctg acc aag act cca gcc aga      974
   Thr Lys Asp Asn Glu His Lys Arg Ser Leu Thr Lys Thr Pro Ala Arg
               290                 295                 300
   aag tct gca cat gtg acc gtg tct ggg ggc acc cca aaa ggc gag gct      1022
   Lys Ser Ala His Val Thr Val Ser Gly Gly Thr Pro Lys Gly Glu Ala
           305                 310                 315
   gtg ctt ggg aca cac aaa tta aag acc atc acg ggg aat tct gct gct   1070
   Val Leu Gly Thr His Lys Leu Lys Thr Ile Thr Gly Asn Ser Ala Ala
       320                 325                 330
   gtt att acc cca ttc aag ttg aca act gag gca acg cag act cca gtc   1118
   Val Ile Thr Pro Phe Lys Leu Thr Thr Glu Ala Thr Gln Thr Pro Val
   335                 340                 345                 350
   tcc aat aag aaa cca gtg ttt gat ctt aaa gca agt ttg tct cgt ccc   1166
   Ser Asn Lys Lys Pro Val Phe Asp Leu Lys Ala Ser Leu Ser Arg Pro
                   355                 360                 365
   ctc aac tat gaa cca cac aaa gga aag cta aaa cca tgg ggg caa tct   1214
   Leu Asn Tyr Glu Pro His Lys Gly Lys Leu Lys Pro Trp Gly Gln Ser
               370                 375                 380
   aaa gaa aat aat tat cta aat caa cat gtc aac aga att aac ttc tac   1262
   Lys Glu Asn Asn Tyr Leu Asn Gln His Val Asn Arg Ile Asn Phe Tyr
           385                 390                 395
   aag aaa act tac aaa caa ccc cat ctc cag aca aag gaa gag caa cgg   1310
   Lys Lys Thr Tyr Lys Gln Pro His Leu Gln Thr Lys Glu Glu Gln Arg
       400                 405                 410
   aag aaa cgc gag caa gaa cga aag gag aag aaa gca aag gtt ttg gga   1358
   Lys Lys Arg Glu Gln Glu Arg Lys Glu Lys Lys Ala Lys Val Leu Gly
   415                 420                 425                 430
   atg cga agg ggc ctc att ttg gct gaa gat taa taatttttta acatcttgta 1411
   Met Arg Arg Gly Leu Ile Leu Ala Glu Asp
                   435                 440
   aatattcctg tattctcaac ttttttcctt ttgtaaattt ttttttttgc tgtcatcccc 1471
   actttagtca cgagatcttt ttctgctaac tgttcatagt ctgtgtagtg tccatgggtt 1531
   cttcatgtgc tatgatctct                                             1551
<210> 4
   <211> 440
   <212> PRT
   <213> Homo sapiens
<400> 4
   Met Ile Ile Pro Ser Leu Glu Glu Leu Asp Ser Leu Lys Tyr Ser Asp
    1               5                  10                  15
   Leu Gln Asn Leu Ala Lys Ser Leu Gly Leu Arg Ala Asn Leu Arg Ala
               20                  25                  30
   Thr Lys Leu Leu Lys Ala Leu Lys Gly Tyr Ile Lys His Glu Ala Arg
           35                  40                  45
   Lys Gly Asn Glu Asn Gln Asp Glu Ser Gln Thr Ser Ala Ser Ser Cys
       50                  55                  60
   Asp Glu Thr Glu Ile Gln Ile Ser Asn Gln Glu Glu Ala Glu Arg Gln
   65                  70                  75                  80
   Pro Leu Gly His Val Thr Lys Thr Arg Arg Arg Cys Lys Thr Val Arg
                   85                  90                  95
   Val Asp Pro Asp Ser Gln Gln Asn His Ser Glu Ile Lys Ile Ser Asn
               100                 105                 110
   Pro Thr Glu Phe Gln Asn His Glu Lys Gln Glu Ser Gln Asp Leu Arg
           115                 120                 125
   Ala Thr Ala Lys Val Pro Ser Pro Pro Asp Glu His Gln Glu Ala Glu
       130                 135                 140
   Asn Ala Val Ser Ser Gly Asn Arg Asp Ser Lys Val Pro Ser Glu Gly
   145                 150                 155                 160
   Lys Lys Ser Leu Tyr Thr Asp Glu Ser Ser Lys Pro Gly Lys Asn Lys
                   165                 170                 175
   Arg Thr Ala Ile Thr Thr Pro Asn Phe Lys Lys Leu His Glu Ala His
               180                 185                 190
   Phe Lys Glu Met Glu Ser Ile Asp Gln Tyr Ile Glu Arg Lys Lys Lys
           195                 200                 205
   His Phe Glu Glu His Asn Ser Met Asn Glu Leu Lys Gln Pro Ile Asn
       210                 215                 220
   Lys Gly Gly Val Arg Thr Pro Val Pro Pro Arg Gly Arg Leu Ser Val
   225                 230                 235                 240
   Ala Ser Thr Pro Ile Ser Gln Arg Arg Ser Gln Gly Arg Ser Cys Gly
                   245                 250                 255
   Pro Ala Ser Gln Ser Thr Leu Gly Leu Lys Gly Ser Leu Lys Pro Ser
               260                 265                 270
   Ala Ile Ser Ala Ala Lys Thr Gly Val Arg Phe Ser Ala Ala Thr Lys
           275                 280                 285
   Asp Asn Glu His Lys Arg Ser Leu Thr Lys Thr Pro Ala Arg Lys Ser
       290                 295                 300
   Ala His Val Thr Val Ser Gly Gly Thr Pro Lys Gly Glu Ala Val Leu
   305                 310                 315                 320
   Gly Thr His Lys Leu Lys Thr Ile Thr Gly Asn Ser Ala Ala Val Ile
                   325                 330                 335
   Thr Pro Phe Lys Leu Thr Thr Glu Ala Thr Gln Thr Pro Val Ser Asn
               340                 345                 350
   Lys Lys Pro Val Phe Asp Leu Lys Ala Ser Leu Ser Arg Pro Leu Asn
           355                 360                 365
   Tyr Glu Pro His Lys Gly Lys Leu Lys Pro Trp Gly Gln Ser Lys Glu
       370                 375                 380
   Asn Asn Tyr Leu Asn Gln His Val Asn Arg Ile Asn Phe Tyr Lys Lys
   385                 390                 395                 400
   Thr Tyr Lys Gln Pro His Leu Gln Thr Lys Glu Glu Gln Arg Lys Lys
                   405                 410                 415
   Arg Glu Gln Glu Arg Lys Glu Lys Lys Ala Lys Val Leu Gly Met Arg
               420                 425                 430
   Arg Gly Leu Ile Leu Ala Glu Asp
           435                 440